# EUROPEAN PATENT APPLICATION

(11) **EP 4 289 433 A1**
(43) Date of publication of application: **13.12.2023**
(21) Application number: 22177795.6
(22) Date of filing: 08.06.2022
(51) Int. Cl.: A61K 31/663, A61P 9/00, A61P 25/00

(54) **PREVENTION OF SYMPATHETIC DENERVATION OF THE PINEAL GLAND IN CARDIAC DISEASE**

(71) Applicant: Technische Universität München, 80333 München (DE)
(72) Inventor: ENGELHARDT, Stefan, 80802 Munich (DE); ZIEGLER, Karin, 80802 Munich (DE)
(74) Representative: Weickmann & Weickmann PartmbB

(57) **Abstract**

The present invention relates the use of an anti-inflammatory agent for inhibiting and/or preventing damage of a peripheral ganglion in a subject, particularly in a subject suffering from a cardiac disease. The present invention also relates to the use of an anti-inflammatory agent for preserving cardiac innervation in a subject suffering from cardiac disease. Further, the present invention relates to method for identifying a subject being at risk of damage of a peripheral ganglion, comprising determining in said subject at least one parameter associated with a risk of damage ganglion damage, wherein the parameter may be a ganglion-specific biomarker. Furthermore, the invention relates to the use of a modulator of at least one of said ganglion-specific biomarkers for preventing damage of a peripheral ganglion in a subject.

## Description

The present invention relates the use of an anti-inflammatory agent for inhibiting and/or preventing damage of a peripheral ganglion in a subject, particularly in a subject suffering from a cardiac disease. The present invention also relates to the use of an anti-inflammatory agent for preserving cardiac innervation in a subject suffering from cardiac disease. Further, the present invention relates to method for identifying a subject being at risk of damage of a peripheral ganglion, comprising determining in said subject at least one parameter associated with a risk of damage ganglion damage, wherein the parameter may be a ganglion-specific biomarker. Furthermore, the invention relates to the use of a modulator of at least one of said ganglion-specific biomarkers for preventing damage of a peripheral ganglion in a subject.

### BACKGROUND

In healthy humans, the sleep-wake cycle is tightly controlled by the daytime-dependent diurnal secretion of melatonin that achieves synchrony with the earth's 24-hour day and night (diurnal) cycle (1-3). Melatonin synthesis occurs in the pineal gland and is, together with its secretion, tightly controlled by sympathetic neurons which project from the superior cervical ganglia (SCG). Next to pineal gland-innervating neurons, the SCG harbors also heart-innervating neurons (4, 5). The end organ-innervating neurons in the SCG receive input from spinal preganglionic neurons, which are regulated by descending spinal projections from central sympathetic nuclei (6). In heart disease, overactivation of the sympathetic nervous system occurs and is recognized as a key pathophysiological factor (7, 8).

Much less appreciated, but likewise frequently occurring in cardiac disease, are disruptions of sleep-wake rhythmicity (9). These sleep abnormalities considerably contribute to the overall disease burden and there is no consensus as to their treatment (*10*). The loss of diurnal rhythmicity in cardiac disease is accompanied by impaired pineal gland function suggested by low melatonin levels (11, 12). It has remained elusive till date, which mechanisms underly the altered sleep-wake cycle in cardiac disease and the role of pineal gland innervation has not been addressed.

### SUMMARY OF THE INVENTION

It was an object of the invention to inhibit the occurrence of damages, e.g., degeneration and/or death of cells, in peripheral ganglia, particularly in the superior cervical ganglion. It was a further object of the invention to preserve cardiac innervation in a subject suffering from a cardiac disease. It was a further object of the invention to prevent disturbances of the sleep-wake cycle in a subject suffering from a cardiac disease. It was still a further object of the invention to identify a subject in risk of the occurrence of damages, e.g., degeneration and/or death of cells, in peripheral ganglia, particularly in the superior cervical ganglion.

The present inventors charted the pineal gland-controlling neuronal circuits in cardiac disease. The results indicate severe and possibly irreversible immune-mediated destruction of a specific subset of sympathetic neurons that control diurnal rhythmicity of pineal melatonin. These data elucidate defective sympathetic control of the pineal gland to underlie the disturbance of circadian rhythmicity in cardiovascular disease.

In view of these findings, the present application relates to are several aspects of clinical relevance.

A first aspect of the invention relates to the administration of an anti-inflammatory agent for use in a method for inhibiting and/or preventing damage of a peripheral ganglion, particularly in the superior cervical ganglion in a subject. In certain embodiments, the damage may comprise degeneration and/or death of cells in said peripheral ganglion, for example, degeneration and/or death of neuronal cells.

A further aspect of the invention relates to the administration of an anti-inflammatory agent for use in the treatment of a subject suffering from a disorder, particularly a cardiac disease associated with and/or accompanied by cell degeneration and/or cell death in a peripheral ganglion, for example, degeneration and/or death of neuronal cells.

A further aspect of the invention relates to the administration of an anti-inflammatory agent for use in a method for preserving cardiac innervation in a subject suffering from a cardiac disease.

The above aspects are based on the observation of fibrotic remodeling in peripheral ganglions of subjects suffering from a cardiac disease. This fibrotic remodeling can be presumably regarded as a sign of irreversible tissue damage. If detected at early stages of ganglionic damage, the application of a preventive, anti-inflammatory intervention will protect patients from damage of their autonomic ganglion structure and/or function.

A further aspect of the invention is to provide a method for identifying a subject being at risk of damage of a peripheral ganglion, comprising determining in said subject at least one parameter associated with damage or risk of damage of a peripheral ganglion. The parameter may be selected from a physical parameter such as an aberrant periodic repolarization dynamics, and ganglion size, and/or a physiological parameter such as ganglion function, inflammation and/or an abnormal value of a biomarker, e.g., a biomarker generally associated with a cardiac disease, and/or a biomarker specifically associated with damage of a peripheral ganglion. Particularly, this method is suitable for identifying a cardiac patient that is at risk for pineal denervation and/or sleep disorder.

Still a further aspect of the invention is the use of a modulator, e.g., an agonist or an antagonist, of at least one of said ganglion-specific biomarkers for preventing damage of a peripheral ganglion in such a subject.

Still a further aspect of the invention relates to the use of a melatonin receptor agonist in order to prevent and/or ameliorate disturbances of the sleep-wake cycle in a subject suffering from a cardiac disease, particularly in a subject suffering from a cardiac disease associated with damage in a peripheral ganglion.

### PREFERRED EMBODIMENTS

In the following, preferred embodiments of the specification are listed:
1. An anti-inflammatory agent for use in a method for inhibiting and/or preventing damage of a peripheral ganglion in a subject.
2. An anti-inflammatory agent for the use of embodiment 1, wherein the damage comprises degeneration and/or death of cells in said peripheral ganglion.
3. An anti-inflammatory agent for the use of embodiment 1 or 2, wherein the damage comprises degeneration and/or death of neuronal cells optionally accompanied by degeneration and/or death of glial cells and/or fibrotic scarring.
4. An anti-inflammatory agent for the use of any one of embodiments 1-3, wherein the damage comprises fibrotic remodeling of the peripheral ganglion.
5. An anti-inflammatory agent for the use of any one of embodiments 1-4, wherein the subject is suffering from a disorder associated with and/or accompanied by degeneration and/or cell death in a peripheral ganglion.
6. An anti-inflammatory agent for the use of any one of embodiments 1-5, wherein the subject is suffering from a disorder associated with and/or accompanied by degeneration and/or death of neuronal cells in a peripheral ganglion.
7. An anti-inflammatory agent for the use of any one of embodiments 1-6, wherein the subject is suffering from a disorder selected from a cardiac disease, obesity, prostate cancer, familial dysautonomia, autonomic neuropathy, diabetes, rheumatic arthritis, and a gastro-intestinal tract infection optionally associated with a sympathetic dysfunction and/or linked to a neuroinflammatory cause.
8. An anti-inflammatory agent for the use of embodiment 7, wherein the subject is suffering from a cardiac disease.
9. An anti-inflammatory agent for the use of embodiment 8, wherein the cardiac disease is selected from hypertension, myocardial infarction, congestive heart failure, cardiac arrhythmia, coronary artery disease or pressure overload-induced cardiac dysfunction.
10. An anti-inflammatory agent for the use of any one of embodiments 1-6, wherein the subject is suffering from a disorder selected from age-dependent pineal gland denervation, atrophy, dysfunctional melatonin synthesis and pineal gland calcification.
11.An anti-inflammatory agent for the use of embodiment 6-10, wherein the disorder is associated with a sleep disturbance.
12.An anti-inflammatory agent for the use of any one of embodiments 6-11, wherein the subject is suffering from an acute form of the disorder.
13. An anti-inflammatory agent for the use of embodiment 12, wherein the subject is suffering from an acute myocardial infarction, and wherein the anti-inflammatory agent is administered in addition to a standard treatment, particularly to a percutaneous revascularization treatment (PCA), e.g., a revascularization treatment of the left anterior descending artery, or wherein the subject is suffering from a pressure overload-induced cardiac dysfunction, and wherein the anti-inflammatory agent is administered in addition to a standard treatment.
14.An anti-inflammatory agent for the use of any one of embodiments 1-13, wherein the anti-inflammatory agent is administered as a preventive intervention.
15.An anti-inflammatory agent for the use of any one of embodiments 1-14, wherein the anti-inflammatory agent is administered when the peripheral ganglion is still substantially intact.
16. An anti-inflammatory agent for the use any one of embodiments 1-15, wherein the anti-inflammatory agent is administered when the peripheral ganglion is characterized by the absence of ganglion hypertrophy, disrupted ganglion activity and/or ganglion inflammation.
17.An anti-inflammatory agent for the use of embodiment 16, wherein ganglion hypertrophy is indicated by an increased ganglion size, e.g., assessed by imaging such as ultrasound, wherein disrupted ganglion activity is indicated by increased periodic repolarization dynamics, e.g., assessed by 3D high-resolution resting ECGs and/or by a decreased blood melatonin level, and wherein ganglion inflammation is indicated by an increased number of immune cells, e.g., macrophages.
18. An anti-inflammatory agent for the use of any one of embodiments 1-17, wherein the peripheral ganglion is a heart innervating ganglion, particularly the superior cervical ganglion (SCG) and/or the stellate ganglion.
19.An anti-inflammatory agent for the use of embodiment 18, wherein the peripheral ganglion is the superior cervical ganglion (SCG).
20.An anti-inflammatory agent for the use of any one of embodiments 1-19, wherein the anti-inflammatory agent is locally administered to the peripheral ganglion, e.g., by injection.
21.An anti-inflammatory agent for the use of any one of embodiments 1-20, wherein the subject is identified as being at risk of damage of a peripheral ganglion.
22.An anti-inflammatory agent for the use of embodiment 21, wherein the risk of damage of a peripheral ganglion is identified by determining at least one parameter selected from periodic repolarization dynamics, e.g., as assessed by a 3D high-resolution resting ECG, ganglion size, e.g., SCG size, e.g., as assessed by ultrasound, the blood melatonin level, a biomarker associated with cardiac disease including but not limited to ANP, BNP, cTnT and its derivatives and/or a biomarker specifically associated with damage of a peripheral ganglion.
23.An anti-inflammatory agent for the use of any one of embodiments 1-22, wherein the anti-inflammatory agent is selected from:
   - a glucocorticoid, including but not limited to Dexamethasone;
   - an immunoglobulin preparation, including but not limited to IVIg;
   - a complement inhibitor, including but not limited to a C3 antagonist such as Compstatin or Pegcetacoplan, or a C5 antagonist such as Eculizumab, Zilucoplan, Tesidolumab, or SKY59;
   - a nucleic acid therapeutic, including but not limited to anti-miR21;
   - an immunomodulator, including but not limited to Alemtuzumab, Mitoxantron, or Orcrelizumab;
   - an antagonist of a chemokine, cytokine or colony-stimulating factor including but not limited to Etanercept, Adalimumab or PLX5622; and
   - a macrophage inhibitor including but not limited to a bisphosphonate compound such as clodronate.
24. An anti-inflammatory agent for the use of any one of embodiments 1-23, further comprising administration of melatonin.
25.An anti-inflammatory agent for the use of any one of embodiments 1-24, wherein the subject is a human subject.
26.A method for identifying a subject being at risk of damage of a peripheral ganglion, comprising determining at least one parameter selected from a physical parameter such as periodic repolarization dynamics, e.g., as assessed by a 3D high-resolution resting ECG, and ganglion size, e.g., SCG size, e.g., as assessed by ultrasound, and/or a physiological parameter such as ganglion function, inflammation and/or an abnormal value of a biomarker, e.g., melatonin, a biomarker generally associated with a cardiac disease, and/or a biomarker specifically associated with damage of a peripheral ganglion.
27. The method of embodiment 26, wherein the biomarker is a biomarker generally associated with a cardiac disease including but not limited to ANP, BNP, cTnT and derivatives thereof.
28.The method of embodiment 27, wherein the biomarker is a biomarker specifically associated with damage or risk of damage of a peripheral ganglion.
29. The method of embodiment 28, wherein the ganglion-specific biomarker is selected from a gene which is characterized by an altered, e.g., increased or decreased expression level associated with damage or risk of damage in a peripheral ganglion.
30. A compound which is a modulator of a biomarker selected from a gene which is characterized by an altered, e.g., increased, or decreased expression level associated with damage or risk of damage in a peripheral ganglion for use in a method for preventing damage of a peripheral ganglion in a subject.
31. The compound of embodiment 30 for the use of embodiment 30, which is an agonist of the biomarker.
32. The compound of embodiment 30 for the use of embodiment 30, which is an antagonist of the biomarker.
33. The compound of any one of embodiment 30-32 for the use of embodiment 30, which is selected from an antibody or an antigen-binding fragment thereof and a nucleic acid molecule, e.g., an mRNA molecule, an antisense molecule, or a siRNA molecule.
34. A melatonin receptor agonist in order to prevent and/or ameliorate disturbances of the sleep-wake cycle in a subject suffering from a cardiac disease, particularly in a subject suffering from a cardiac disease associated with damage in a peripheral ganglion.

### DETAILED DESCRIPTION

The present inventors have performed immunostaining of sympathetic axons in optically cleared pineal glands obtained by autopsy from humans and from mice with cardiac disease and found their massive denervation compared to controls. Single cell, nuclear and bulk RNA sequencing traced this defect back to the peripheral ganglia, particularly the superior cervical ganglion, which responded to cardiac disease by accumulation of inflammatory macrophages and the selective loss of pineal gland-innervating neurons. Primary cell co-cultures recapitulated macrophage-mediated neuronal cell death. Supplementation of the effector hormone melatonin rescued diurnal rhythmicity in mice subjected to surgical denervation of the pineal gland. These data suggest a pathophysiological paradigm, where autonomic ganglia act as a 'relay station' to connect a locally confined disease to anatomically distant organs and effects.

Based on these data it is expected that administration of an anti-inflammatory agent will be clinically effective in inhibiting and/or preventing damage of a peripheral ganglion, wherein the damage may be caused by and/or associated with neuronal degeneration and/or cell death.

In certain embodiments, the damage comprises degeneration and/or death of cells in said peripheral ganglion, particularly degeneration and/or death of neuronal cells. In certain embodiments, the damage also affects glial cells and/or results in fibrotic scarring. Pathological adaptations regarding the glial cell population hereby include but are not limited to proliferation of myelinating subpopulations and/or increased secretion of proinflammatory cytokines.

In certain embodiments, the damage comprises a ganglionic fibrosis, particularly a fibrotic remodeling of the peripheral ganglion optionally associated with ganglion hypertrophy, i.e., a size increase of the ganglion. Fibrotic remodeling in a peripheral ganglion is typically characterized by fibrotic scarring and/or replacement of functional neuronal cells by fibroblasts and/or glial cells resulting in loss, in many cases irreversible loss of neuronal function.

For clinical use, particularly in preventive applications, the anti-inflammatory agent is administered in an effective dose to a subject suffering from a disorder associated with and/or accompanied by degeneration and/or cell death in a peripheral ganglion. The subject can be any mammalian subject, typically a human subject.

In certain embodiments, the subject is suffering from a disorder selected from a cardiac disease, obesity, prostate cancer, familial dysautonomia, autonomic neuropathy, diabetes, rheumatic arthritis, and a gastro-intestinal tract infection optionally associated with a sympathetic dysfunction and/or linked to a neuroinflammatory cause. In particular embodiments, the subject is suffering from a cardiac disease, wherein the cardiac disease may be selected from hypertension, myocardial infarction, congestive heart failure, cardiac arrhythmia, or coronary artery disease.

In certain embodiments, the subject is suffering from a disorder selected from age-dependent pineal gland denervation, atrophy, dysfunctional melatonin synthesis and pineal gland calcification.

In certain embodiments, the cardiac disease is associated with sleep disturbance, e.g., associated with a reduced melatonin level in the subject's blood.

Administration of the anti-inflammatory agent according to the present invention is particularly useful when the subject is suffering from an acute form of the disorder, e.g., when signs and/or symptoms of the disease are observable for a time period of up to 4 weeks, up to 1 week or up to 1 day.

The anti-inflammatory agent may be administered as a monotherapy or in combination with a further medicament, particularly a medicament suitable for the treatment of the disorder underlying the risk of ganglion damage. For example, the anti-inflammatory agent may be administered with any standard treatment, e.g., standard surgical procedure and/or standard medicament useful for the treatment of respective disorder. In certain embodiments, the anti-inflammatory agent may be administered with any standard treatment for a cardiac disease, e.g., a standard surgical procedure and/or standard medicament useful for the treatment of the respective cardiac disease.

In certain embodiments, the subject is suffering from an acute myocardial infarction, and the anti-inflammatory agent is administered in addition to a standard treatment, particularly in addition to a percutaneous revascularization treatment (PCA), e.g., a revascularization treatment of the left anterior descending artery. Other possible indication fields include but are not limited to a pressure overload-induced cardiac dysfunction, e.g., acute sympathetic dysfunction during hypertensive crisis and/or acute cardiac tachyarrhythmia.

In certain embodiments, the anti-inflammatory agent is administered as a preventive intervention. In these embodiments, the subject has a substantially intact peripheral ganglion that, however, may be at risk of suffering damage.

In particular embodiments, the peripheral ganglion may be characterized by a substantially normal ganglion size, e.g., absence of ganglion hypertrophy. Ganglion hypertrophy may be characterized by an increase in ganglion size (measured as length and/or volume) of not more than about 50% or of not more than about 25%. Hypertrophy may be assessed by imaging such as ultrasound.

In further particular embodiments, the peripheral ganglion may be characterized by substantially normal ganglion activity, e.g., absence of disrupted activity. Disrupted activity may be characterized by increased periodic repolarization dynamics, e.g., assessed by 3D high-resolution resting ECGs and/or by a decreased blood melatonin level and/or inflammation at the start of treatment.

In further particular embodiments, the peripheral ganglion may be characterized by absence of inflammation. Absence of inflammation may be characterized by increased inflammation parameters such as an increased number of pro-inflammatory immune cells, e.g., macrophages, as determined e.g., by molecular imaging modalities, including PET, MRI and optoacoustic methods (e.g., inflammation score).

The peripheral ganglion may be any ganglion in the body, e.g., the human body, located outside the brain and spinal cord. For example, the peripheral ganglion may be selected from the sympathetic trunk, dependent on the disease context, e.g., for heart disease a cardiac-innervating ganglion such as the superior cervical ganglion (SCG) and/or the stellate ganglion. In particular embodiments, the peripheral ganglion is the SCG.

The anti-inflammatory agent is typically administered to the subject as a pharmaceutical composition comprising the active agent and a pharmacologically acceptable carrier. The carrier may be any suitable pharmaceutical carrier. The anti-inflammatory agent is administered in a therapeutically effective dose which may be determined by the skilled practitioner. The pharmaceutical composition may be in the form of a solid dosage form, e.g., a tablet, a capsule etc. or a liquid dosage form, e.g., a solution, emulsion, suspension, or the like. In particular embodiments, the pharmaceutical composition is a liquid dosage form adapted for injection.

The anti-inflammatory agent or the pharmaceutical composition may be administered in any suitable way, e.g., systemically, or locally. Systemical administration may include oral, parenteral, e.g., by subcutaneous injection, or intravenous infusion or injection, and transmucosal application. In certain embodiments, a local administration is preferred. Local administration may include injection of the anti-inflammatory agent or the pharmaceutical composition to a peripheral ganglion.

The anti-inflammatory agent may be any pharmaceutically acceptable anti-inflammatory agent, particularly any pharmaceutically acceptable anti-inflammatory agent capable of counteracting inflammatory process mediated by, and/or associated with pro-inflammatory cells, e.g., pro-inflammatory macrophages.

For example, the anti-inflammatory agent may be a steroidal drug, an immunoglobulin preparation, an inhibitor of the complement cascade, an immunomodulator, and/or an antagonist of a chemokine, cytokine, or colony-stimulating factor. In certain embodiments, the anti-inflammatory agent is a biological, e.g., a monoclonal antibody, a recombinant fusion protein or a nucleic acid therapeutic.

In certain embodiments, the anti-inflammatory agent may be selected from:
- a glucocorticoid, including but not limited to Dexamethasone;
- an immunoglobulin preparation, including but not limited to IVIg;
- a complement inhibitor, including but not limited to a C3 antagonist such as Compstatin or Pegcetacoplan, or a C5 antagonist such as Eculizumab, Zilucoplan, Tesidolumab, or SKY59;
- a nucleic acid therapeutic including but not limited to anti-miR21;
- an immunomodulator, including but not limited to Alemtuzumab, Mitoxantron, or Orcrelizumab; and
- an antagonist of a chemokine, cytokine or colony-stimulating factor including but not limited to Etanercept, Adalimumab or PLX5622; and
- a macrophage inhibitor including but not limited to a bisphosphonate compound such as clodronate.

In certain embodiments, a macrophage inhibitor such as clodronate is administered locally to a peripheral ganglion, e.g., the SCG, for example to a subject suffering from a pressure overload-induced cardiac dysfunction.

Still a further aspect of the present invention relates to a method for identifying a subject being at risk of damage of a peripheral ganglion. Particularly, this method is suitable for identifying a cardiac patient that is at risk for pineal denervation and/or sleep disorder. The identified subject may then be treated with an anti-inflammatory agent as described above. A subject "being at risk of damage" includes a subject that has not suffered any damage yet but is expected to suffer from damage including complete loss of ganglionic function if left untreated. It also includes a subject that has already suffered a certain degree of damage and is expected to suffer from increased damage including complete loss of ganglionic function if left untreated.

Identification of the subject may comprise determining at least one parameter associated with a risk for ganglion damage. In certain embodiments, the parameter may be a physical parameter such as periodic repolarization dynamics, e.g., as assessed by a 3D high-resolution resting ECG, or ganglion size, e.g., SCG size, e.g., as assessed by ultrasound. An aberrant periodic repolarization dynamics and/or ganglion hypertrophy may be associated with risk of damage.

Further, the parameter may be a physiological parameter such as ganglion function, inflammation and/or a biomarker associated with risk of ganglionic damage. For example, the biomarker may be selected from melatonin, a biomarker generally associated with a cardiac disease, and/or a biomarker specifically associated with damage or risk of damage of a peripheral ganglion. In certain embodiments, a decreased blood melatonin level and/or an abnormal value of a biomarker may be associated with risk of damage. In certain embodiments, the biomarker is a biomarker generally associated with a cardiac disease including but not limited to ANP, BNP, Troponin T and derivatives thereof.

In certain embodiments, the biomarker is specifically associated with damage or risk of damage of a peripheral ganglion, e.g., with damage or risk of damage of the SCG. For example, the ganglion-specific biomarker is a gene, e.g., a human gene, which is characterized by an altered, e.g., increased, or decreased expression level associated with damage or risk of damage in a peripheral ganglion, e.g., the SCG. In certain embodiments, the gene is a gene, e.g., a human gene expressed in pineal gland-innervating neurons. Genes expressed in pineal gland-innervating neurons include tyrosine hydroxylase (*Th*), dopamine-p-hydroxylase (*Dbh*)*,* Neuropeptide Y (*Npy*), peripherin (*Prph*)*,* and Synapsin II (*Syn2*) as typical markers for sympathetic neurons. In addition, genes expressind in pineal gland-innervating neurons include the small nucleolar RNA host gene 11 (*Snhg11*)*,* calcium voltage-gated channel subunit alpha1 A (*Cacna1a*), Ankyrin 2 (*Ank2*)*,* Hand2os1, Semaphorin 6D (*Sema6d*)*,* EPH Receptor A5 (*Epha5*)*,* neuronal cell adhesion molecule (*Nrcam*)*,* and synaptosome associated protein 91 (*Snap91*)*.* Nucleotide sequences of these genes, e.g., human genes, and associated cDNA and transcript sequences, as well as amino acid sequences of polypeptides encoded by these nucleotide sequences can be retrieved from a suitable database such as UniProt (status 01-06-2022). These sequences are incorporated by reference.

Still a further aspect of the present invention relates to a compound which is a modulator of a biomarker selected a gene which is characterized by an altered, e.g., increased or decreased expression level associated with damage or risk of damage in a peripheral ganglion, e.g., the SCG, for use in a method for preventing damage of a peripheral ganglion in a subject. In certain embodiments, the compound may be an agonist of the biomarker, particularly when a decreased expression level is associated with damage or risk of damage in a peripheral ganglion. In certain embodiments, the compound may be an antagonist of the biomarker, particularly when an increased expression level is associated with damage or risk of damage in a peripheral ganglion. In certain embodiments, an agonistic modulator may be the polypeptide including an active fragment or derivative thereof or an mRNA encoding such a polypeptide. In certain embodiments, an antagonistic modulator may be an antibody or an antigen-binding fragment thereof or a nucleic acid molecule, e.g., an mRNA molecule, an antisense molecule, or a siRNA molecule capable of inhibiting gene expression. The modulator may be administered as a pharmaceutical comprising the active agent and a pharmacologically acceptable carrier, e.g., as described above. In certain embodiments, the modulator is administered locally as described above. The modulator may be administered as a monotherapy or in combination with an another medicament, particularly an anti-inflammatory agent as described above.

Still a further aspect of the invention relates to the use of a melatonin receptor agonist, e.g., melatonin or a melatonin analog such as Agomelatin, Ramelteon, or Tasimelteon in order to prevent and/or ameliorate disturbances of the sleep-wake cycle in a subject suffering from a cardiac disease, particularly in a subject suffering from a cardiac disease associated with damage in a peripheral ganglion. The melatonin receptor agonist may be administered as a monotherapy or in combination with an anti-inflammatory agent as described above.

Further, the invention shall be explained in more detail by the following figures and Examples.

### FIGURE LEGENDS

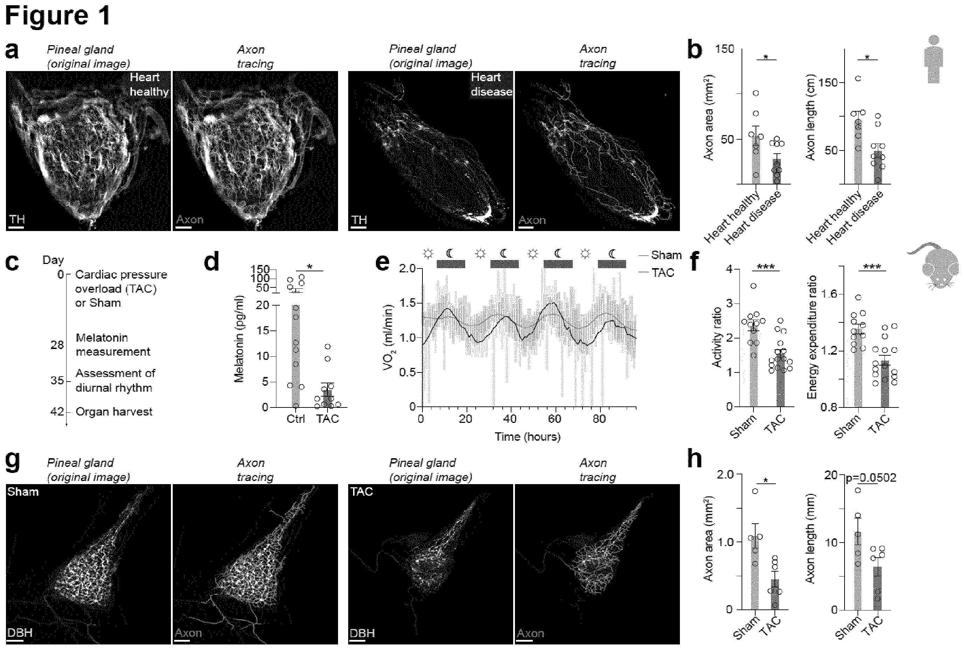

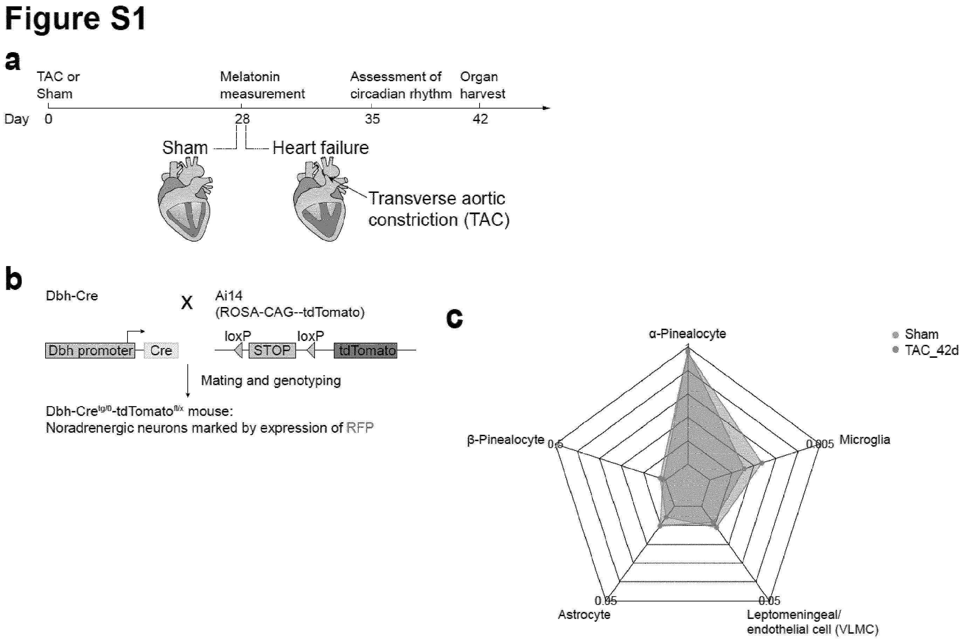

### Figure 1: Cardiac disease causes sympathetic denervation and dysfunction of the pineal gland

(a) Representative images of human pineal glands after clearing and staining for tyrosine hydroxylase (TH). Scale bar: 1 mm. (b) Quantification of axon area and length. (c) Timeline of mouse behavioral study. (d) Measurement of melatonin in plasma from C3H/HeJ mice 28 days after the respective intervention. (e-f) Assessment of diurnal rhythm in C3H/HeJ mice after Sham and TAC. Mouse behavior including metabolic measurements (including oxygen consumption (V_{O2}) and energy expenditure) as well as activity (beam breaks per minute) was continuously recorded. The ratio between dark and light was calculated for activity and energy expenditure (kcal per hour). (g) Representative images of murine pineal glands from dopamine beta-hydroxylase-(DBH) Cre/tdTomato floxed mice 28 days after Sham and TAC. Scale bar: 200 µm. (h) Quantification of axon area and length. Data are mean ± SEM of n=7 (Heart healthy) and n=9 (Heart disease) for quantification of human pineal gland innervation; n=12 (Sham), n=9 (SCGx), and n=10 (TAC) for Melatonin measurements; n=11 (Sham) and n=15 (TAC) for assessment of circadian rhythm; n=5 (Sham) and n=6 (TAC) for quantification of sympathetic axons in murine pineal glands. Student's t-test was applied for statistical analysis of sympathetic innervation, diurnal rhythm (activity, energy expenditure) and plasma melatonin levels. * P< 0.05, *** P<0.001.

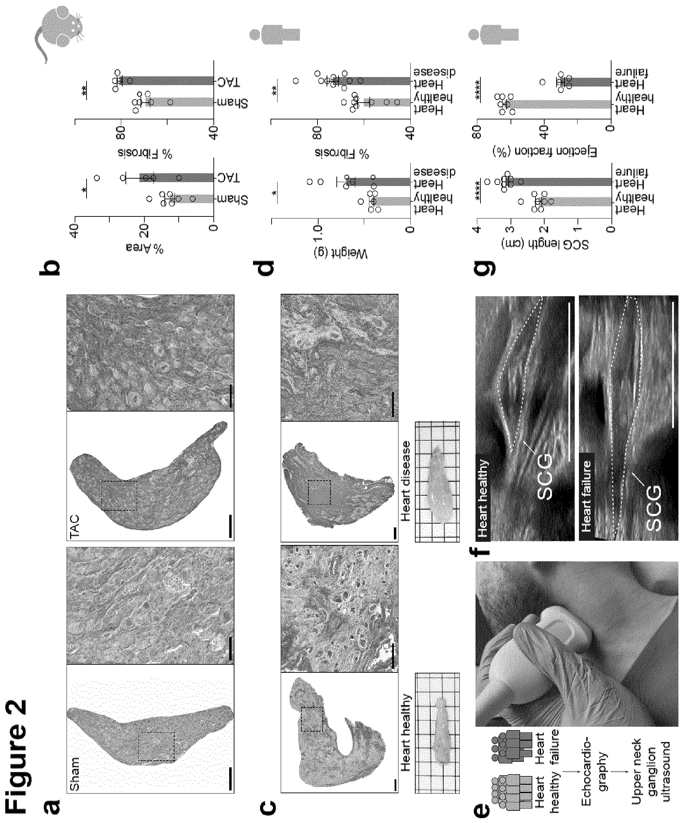

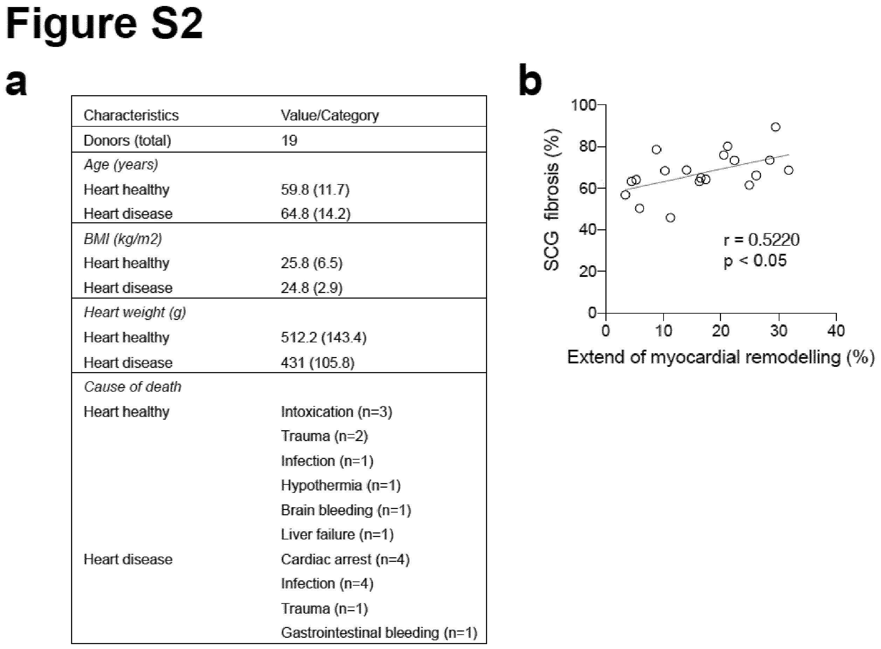

### Figure 2: Chronic cardiac disease causes fibrotic scarring and SCG hypertrophy in mice and humans

(a) *Left* Representative images of murine superior cervical ganglia 42 days after Sham and TAC surgery stained with Fast Green/Sirius Red. Scale bar: 200 µm. *Right* Close-up images of the boxed regions. Scale bar: 50 µm. (b) Quantification of SCG area and fibrosis. (c) *Above left* Paraffin sections stained with Fast Green/Sirius Red. Scale bar: 500 µm. The boxes indicate the region of the close-up images. *Above right* Close-up of the boxed regions. Scale bar: 200 µm. *Below* Representative images of superior cervical ganglia from individuals with and without heart disease. (d) Quantification of SCG weight and Sirius Red-positive area in transverse SCG sections. (e-g) Evaluation of SCG morphometry in a prospective clinical study. (e) *Left* Workflow of the clinical study. *Right* Exemplary setup of an SCG ultrasound examination. (f) Representative images from human SCGs acquired by ultrasound (*left* Heart healthy, *right* Heart failure). Scale bar: 3.5 cm. (f) Quantification of SCG length and ejection fraction. Data are mean ± SEM of n=7 (Sham) and n=5 (TAC) for area and fibrosis; n=6 (Heart healthy) and n=7 (Heart disease) for SCG weight; n=9 (Heart healthy) and n=10 (Heart disease) for fibrosis; n=8 (Heart healthy) and n=6 (Heart failure) for assessment of SCG length ejection fraction. Student's t-test was applied for statistical analysis. * P< 0.05, **P< 0.01, ****P< 0.0001.

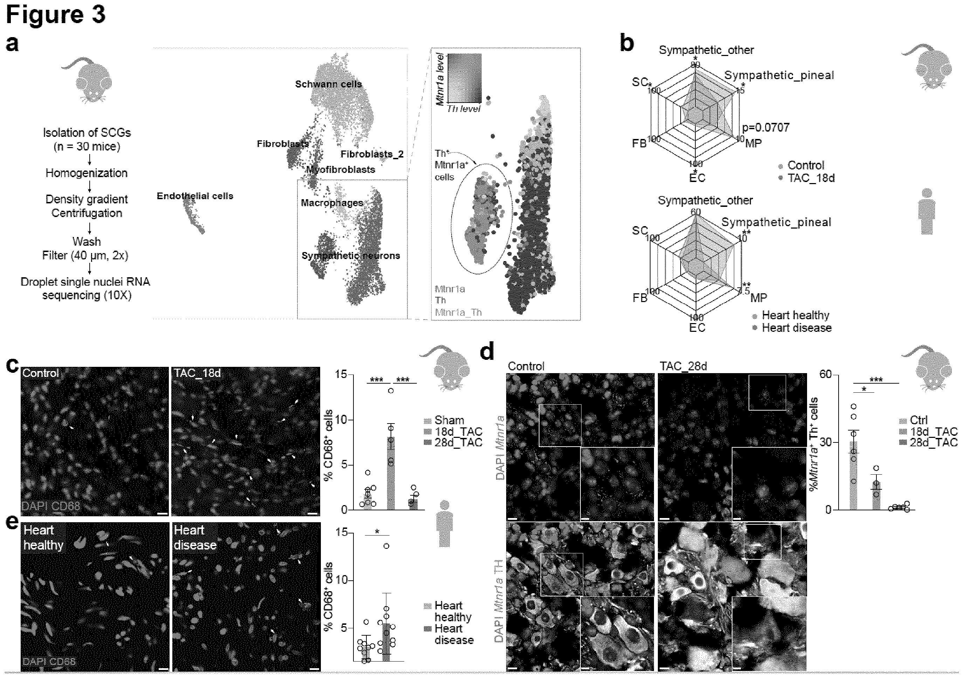

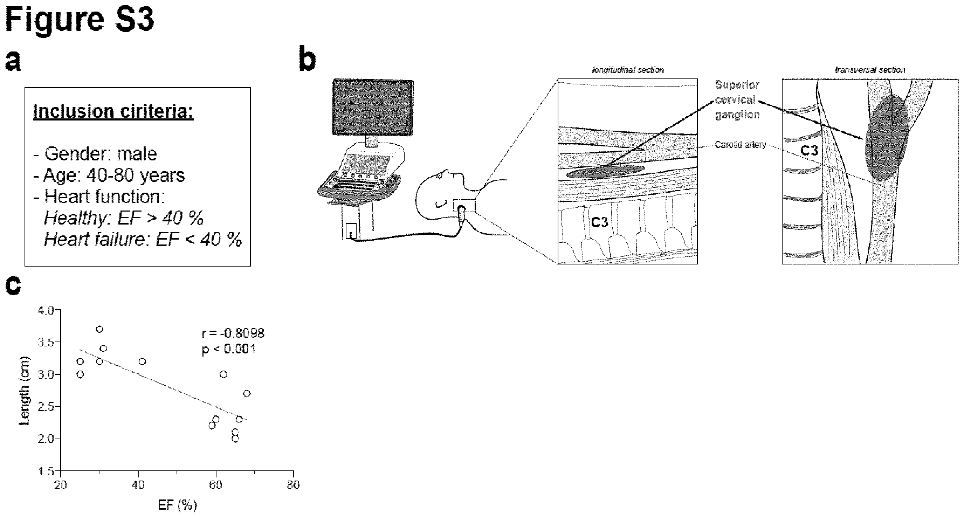

### Figure 3: Heart disease triggers macrophage infiltration and loss of pineal gland-innervating neurons in SCGs.

(a) *Left* Timeline for nuclei (snSeq) RNA seq experiments. *Middle* UMAP projection of 9,172 cellular nuclei from SCGs isolated 5 days after Sham and cardiac pressure overload (TAC). *Right* (Co-)Expression of melatonin receptor 1A (*Mtnr1a*) and tyrosine hydroxylase (*Th*) in the different subclusters of sympathetic neurons in the UMAP representation as indicated by the color scale. (b) Quantitative assessment of cellular composition in (*above*) mouse and (*below*) human SCGs by genetic deconvolution of RNA sequencing libraries. (c) CD68⁺ cells (arrows) in the respective ganglia with quantification. Scale bar: 10 µm. (d) In situ hybridization and immunofluorescence assays in murine superior cervical ganglia with quantification. The boxes indicate the region of the close-up images. Scale bar: 10 µm (close-up: 5 µm). (e) CD68⁺ cells (arrows) in the respective ganglia with quantification. Scale bar: 10 µm. Data are from n=4 (Control) and n=3 (TAC) for mSCG RNA-seq libraries; n=7 (Sham), n=5 (TAC d18) and n=4 (TAC d28) for quantification of CD68⁺ cells; n=6 (Control, TAC d28) and n=3 (TAC d18) for quantification of *Mtnr1a*⁺ Th⁺ cells; n=3 (Heart healthy; Heart disease) for hSCG RNA-seq libraries; n=9 (Heart healthy) and n=10 (Heart disease) quantification of CD68⁺ cells. Student's t-test was applied for analysis of relative cell fractions (mSCG, hSCG) and of CD68⁺ cells in human SCGs; One-way ANOVA with Bonferroni's post-hoc test was applied for statistical analysis of CD68⁺ and *Mtnr1a*⁺ cells in murine SCGs. * P< 0.05, ** P<0.01, *** P<0.001. Abbreviations: FB - fibroblasts, MP - macrophages, EC - endothelial cells, MyoFB - Myofibroblasts, Sympathetic_pineal - pineal gland-innervating sympathetic neurons, Sympathetic_other - sympathetic neurons innervating other organs, SC - Schwann cells, RBC - red blood cells, TC - T cells, MC - monocytes.

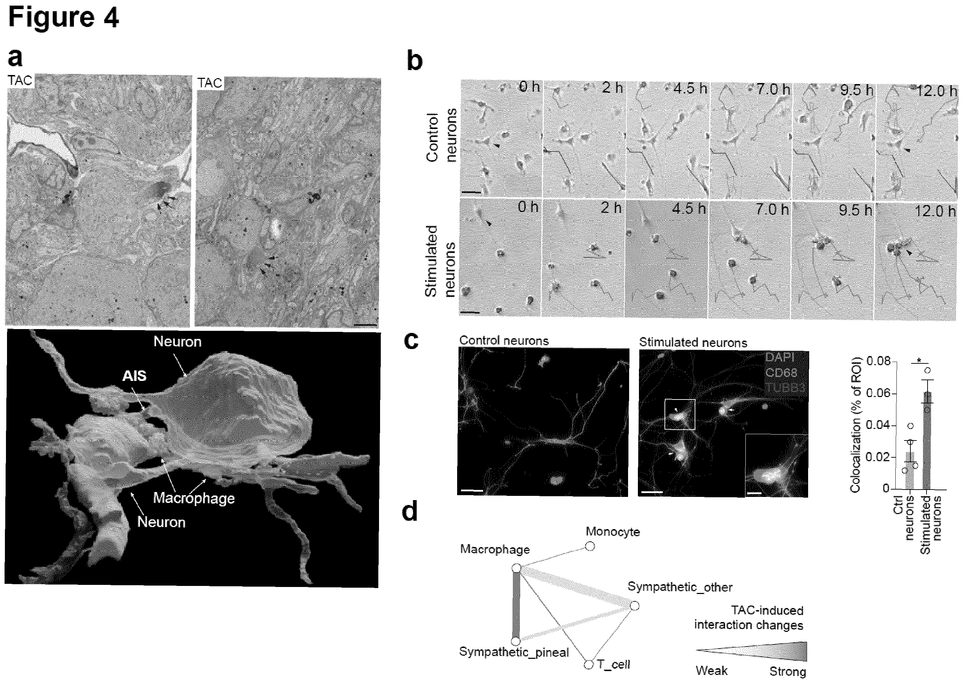

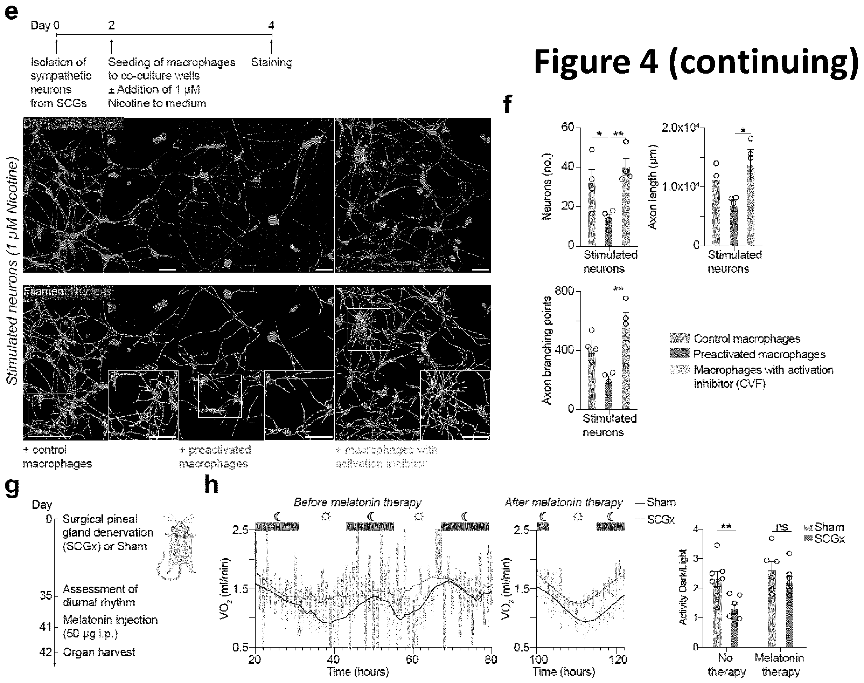

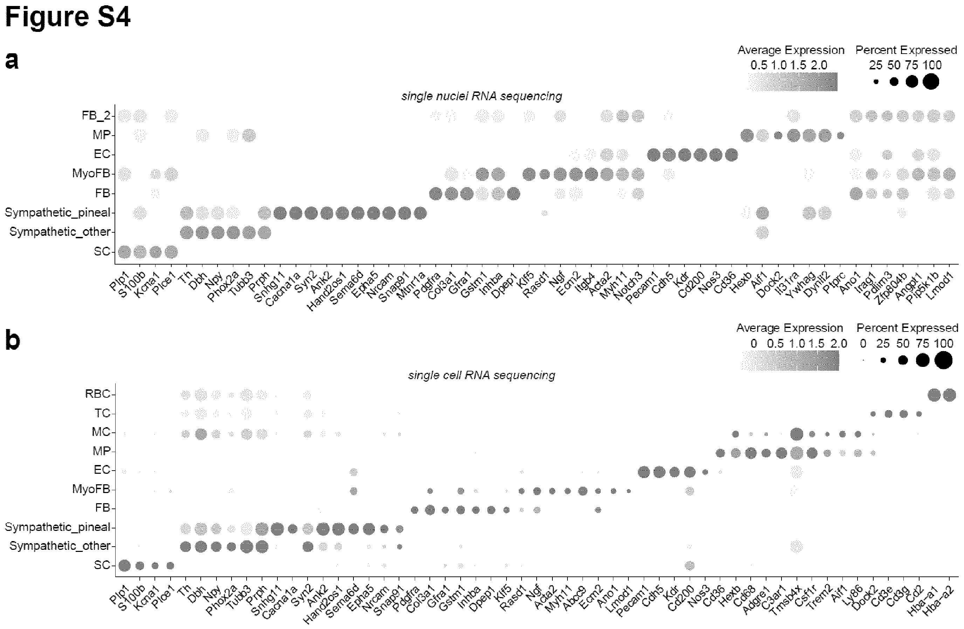

### Figure 4: Loss of pineal gland-innervating neurons is mediated by activated macrophages.

(a) *Above* Representative electron microscopy images 5 days after cardiac pressure overload (TAC) with *below* 3D model. Neurons are labelled blue, macrophages orange. Arrows point to degenerative regions with dark cytoplasm. Scale bar: 5 µm. (b) Representative images from time-lapse series of co-culture between control neurons (*above*) and stimulated neurons (*below*) with preactivated macrophages. Scale bar: 50 µm. (c) Representative images from co-culture of preactivated macrophages and stimulated neurons with quantification of colocalization. Arrows point to spatial proximity between macrophage and axon initiation segment. The boxes indicate the region of the close-up images. Scale bar: 50 µm (close-up: 10 µm). (d) Neuroimmune interaction map in superior cervical ganglia. Relative differences of ligand-receptor pairs between Sham and 5 days after TAC. The color indicates the acute TAC-induced changes in ligand-receptor pairings: white indicates weak, pink middle and red strong. (e) *Above* Experimental strategy for co-culture of adult mouse sympathetic neurons and bone marrow-derived macrophages. *Below* Immunostaining of sympathetic neurons (TUBB3) and macrophages (CD68) in the respective co-culture wells with respective images of filament and nucleus tracing. Scale bar: 50 µm (close-up: 20 µm). (f) Quantification of neuronal cell number (per field of view), axon length and branching points. (g-h) Assessment of diurnal rhythm in C3H/HeJ mice after Sham and bilateral removal of SCG (SCGx). (g) Timeline of the study. From day 35 on, mouse behavior including oxygen consumption (VO2) and activity (beam breaks per minute) was continuously recorded. After 6 days of recording, 50 µg melatonin was once injected intraperitoneally at the time of physiological peak of endogenous melatonin production. (h, *right*) The ratio between dark and light was calculated for activity. Data are mean ± SEM from n=4 independent experiments per group for co-cultivation experiments; n=7 for assessment of diurnal rhythm. Student's t-test was applied for statistical analysis of colocalization; One-way ANOVA with Bonferroni's post-hoc test was applied for statistical analysis of neuronal cell number and axon parameters. Two-way ANOVA with Bonferroni's post-hoc test was applied for statistical analysis of activity. * P< 0.05, ** P<0.01. Abbreviations: CVF - Cobra Venom Factor.

### Figure 5: Inhibition of macrophages by local injection of clodronate.

Ejection fraction 18 days after the respective intervention (Sham; TAC). Two-way ANOVA with Bonferroni's post-hoc test was applied for statistical analysis. * P< 0.05. Abbreviations: TAC - transverse aortic constriction.

### Figure 6: Distribution of axon area and pineal gland size in heart healthy donors by age group.

*Left Axon* area and *right* pineal gland area in the respective age groups.

### Figure S1: Assessment of pineal gland function, innervation and cellular composition.

(a) Timeline for the assessment of pineal gland function. C3H/HeJ mice were subjected to either Sham or TAC (transverse aortic constriction) surgery. After 4 weeks, plasma samples were taken for measurement of melatonin levels. 35 days after the respective intervention, mouse circadian behavior (activity, indirect calorimetry) was continuously assessed for 7 consecutive days. (b) Schematic representation of mouse breeding of dopamin-β-hydroxylase (Dbh)-Cre and Ai14 mice. (c) Genetic deconvolution of murine pineal glands 42 days after surgery. Data are mean from n=2 (Sham) and n=3 (TAC) animals.

### Figure S2: Summary of demographics included in the post-mortem study.

(a) Characteristics of the donors included in the postmortem study. (b) Correlation analysis of SCG fibrosis and extend of myocardial remodelling.

### Figure S3: Trial profile of the prospective clinical study for assessment of SCG dimensions in heart healthy and heart failure individuals.

(a) Inclusion criteria of prospective clinical study for evaluation of SCG length in heart failure. (b) Schematic representation of the SCG location in a longitudinal (*left*) and transversal (*right*) section. At level C3, the landmarks of the longus capitis muscle and internal carotid artery were used to identify the ganglion. (c) Correlation analysis between SCG length and ejection fraction.

### Figure S4: Cell type-specific marker genes for single nuclei and cell RNA sequencing.

Dot plot of cell type-specific genes in single nuclei (a) and cell RNA (b) sequencing. Abbreviations: FB - fibroblasts, MP - macrophages, EC - endothelial cells, MyoFB - Myofibroblasts, Sympathetic_pineal - pineal gland-innervating sympathetic neurons, Sympathetic_other - sympathetic neurons innervating other organs, SC - Schwann cells, RBC - red blood cells, TC - T cells, MC - monocytes.

### Figure S5: Changes of heart function and adrenal morphology after transverse aortic constriction.

(a) Workflow for genetic deconvolution analysis. (b) Timeline of cardiac output (CO) assessed by echocardiography after the respective intervention (Sham, TAC). (c) *Left* Representative images from sections of adrenal glands after Sham and TAC surgery stained with anti-Th antibody and hematoxylin. *Right* Ratio of adrenal medulla to cortex. Scale bar: 2 mm. Data are from Sham n=15 (d0, d5, d18), n=5 (d28) and TAC, n=14 (d0), n=15 (d5), n=18 (d18), n=5 (d28) for cardiac output; n =4-5 per group for assessment of adrenal medulla/cortex ratio. Two-way ANOVA with Bonferroni's post-hoc test was applied for statistical analysis of cardiac output. One-way ANOVA with Bonferroni's post-hoc test was applied for statistical analysis of adrenal medulla. * P<0.05, ** P<0.01, **** P<0.0001.

### Figure S6: Axonal damage of SCG neurons.

(a) Timeline for electron microscopy (EM) experiments. 5 days after the respective intervention, tissue was fixed in situ and harvested for EM. (b) EM analysis of SCGs isolated from mice 5 days after TAC from four independent experiments shows an accumulation of macrophages in close proximity to neurons. The axon initiation segment (AIS) is marked in dark blue (arrow), neurons in light blue and macrophages in orange. Scale bar: 5 µm.

### Figure S7: Macrophage-mediated neuronal death depends on chronic stimulation of co-culture neurons.

(a) *Above* Immunostaining of untreated sympathetic neurons (TUBB3) and macrophages (CD68) in the respective co-culture wells. *Below* Respective images of filament and nucleus tracing. Scale bar: 50 µm (close-up: 20 µm). (b) Quantification of neuronal cell number (per field of view), axon length and branching points. Data are mean ± SEM from n=4 independent experiments per group.

**Figure S8:** Immune-mediated loss of pineal gland-innervating neurons causes loss of diurnal rhythm in cardiac disease. *Left* Schematic representation of the sympathetic circuits under physiological conditions. *Right* Postulated pathological changes in the sympathetic interconnections shown in this paper.

### EXAMPLES

### 1. Materials

| **REAGENT or RESOURCE** | **SOURCE** | **IDENTIFIER** |
|---|---|---|
| **Antibodies** | | |
| Rabbit polyclonal anti-TH | Merck | Cat# 6A2907; RRID: AB_152 |
| Rat anti Mouse CD68 antibody, clone FA-11 | BioRad | MCA1957 |
| CD68 Monoclonal Antibody (KP1), eBioscience^{™} | ThermoFisher/Invitrogen | Cat# 14-0688-82, RRID: AB_11151139 |
| Purified anti-Tubulin Beta 3 (TUBB3) Antibody | BioLegend | BLD-657402, RRID: AB_2562570 |
| Goat polyclonal anti-rabbit, Alexa Fluor 568 | ThermoFisher/Invitrogen | Cat# A-11036, RRID: AB_10563566 |
| Goat polyclonal anti-rabbit, Alexa Fluor Plus 647 | ThermoFisher/Invitrogen | Cat# A-32733, AB_2633282 |
| Goat polyclonal anti-rat, Alexa Fluor 594 | ThermoFisher/Invitrogen | Cat# A-11007, AB_10561522 |
| Donkey polyclonal anti-mouse, Alexa Fluor 488 | ThermoFisher/Invitrogen | Cat# A-21202; RRID: AB_141607 |
| Goat polyclonal anti-mouse, Alexa Fluor 568 | ThermoFisher/Invitrogen | Cat# A-1 1004; RRID: AB_2534072 |
| **Biological Samples** | | |
| Human superior cervical ganglia | Forensic Institute of Ludwig Maximilians University | http:// www.rechtsmedizin.med.unimuenchen.de |
| Human left ventricles | | |
| Human pineal glands | | |
| **Chemicals, Peptides, and Recombinant Proteins** | | |
| RNAscope target retrieval buffer | ACDBio | 322000 |
| RNAscope protease III & IV reagents | ACDBio | 322340 |
| RNAscope probe diluent | ACDBio | 300041 |
| ProLong Gold Antifade Mountant with DAPI | ThermoFisher/Invitrogen | P36935 |
| Paraformaldehyd (reagent grade, crystalline) | Sigma | P6148 |
| HBSS | ThermoFisher/Invitrogen | 14.175.095 |
| PFA (EM-grade) | Science Services | E15710 |
| Glutaraldehyde (EM-grade) | Science Services | E16220 |
| Cacodylate buffer | Science Services | E11654 |
| Calcium chloride | Roth | CN93.1 |
| Homogenization buffer | -- (made in house) | -- |
| Diluent | -- (made in house) | -- |
| Working solution | -- (made in house) | -- |
| 30% OptiPrep | -- (made in house) | -- |
| 40% OptiPrep | -- (made in house) | -- |
| Phenol Red | Merck | P5530 |
| Albumin Fraction V | Roth | 8076.4 |
| Trypane blue | ThermoFisher/Invitrogen | T10282 |
| 4',6-Diamidino-2-phenylindol -dihydrochlorid (DAPI) | Merck | D9542 |
| SYTOX green | ThermoFisher/Invitrogen | S7020 |
| RNAse-Free DNase | QIAGEN | 1023460 |
| Trition X-100 (laboratory grade) | Sigma Aldrich | 9002-93-1 |
| PBS (sterile) PBS (non-sterile) | Life Technologies Roth | 14190-169 1111.2 |
| Vectashield Antifade Mounting Medium H-1000 | Biozol | VEC-H-1000 |
| Corning^{®} Laminin | VWR | 734-1098 |
| Collagenase II | Worthington/Cellsystems | LS004176 |
| Albumin human | Merck | A9731 |
| ACSF | -- (made in house) | -- |
| NGF 2.5S Protein, mouse | Merck | 01-125 |
| Gibco Neurobasal A-Medium | ThermoFisher/Invitrogen | 10888022 |
| Fetal bovine serum | Merck | F7524 |
| Penicillin/Streptomycin | ThermoFisher/Invitrogen | 15070063 |
| Supplement B27 | Life Technologies | 17504044 |
| Recombinant Murine M-CSF | Peprotech | AF-315-02 |
| Lipopolysaccharides from Escherichia coli O55:B5 | Merck | L4524 |
| Cobra Venom Factor | Quidel | A600 |
| (-)-Nicotine | Sigma | N3876 |
| Sodium acide | Sigma | S-2002 |
| Fast Green FCF | Merck | F7252 |
| Direct Red 80 | Merck | 365548 |
| DAB Substrate | Abcam | ab64238 |
| X-CLARITY Hydrogel Solution Kit (Hydrogel Solution, Polymerization Initiator) | OLS | C1310X |
| Electrophoretic Tissue Clearing Solution | OLS | C30001 |
| X-CLARITY Mounting Solution | OLS | C13102 |
| **Oligonucleotides** | | |
| Mm-Mtnr1a C3 probe for RNAScope | ACDBio | 403601-C3 |
| **Critical Commercial Assays** | | |
| RNeasy mini-prep kit | QIAGEN | 74104 |
| RNeasy midi-prep kit | QIAGEN | 75144 |
| RNAScope fluorescent multiplex detection reagent kit | ACDBio | 320850 |
| Melatonin ELISA | Abcam | ab213978 |
| Ethyl acetate | Sigma | 270989 |
| Heparin | Braun | 2540309 |
| Chromium Next GEM Single Kit v3.1 | 10X | 1000127 |
| TruSeq Stranded mRNA | Illumina | 20020595 |
| **Deposited Data** | | |
| Raw and analyzed data | This paper | |
| **Experimental Models: Organisms/Strains** | | |
| Mouse: BI6 C57 N | In house breeding | N/A |
| Mouse: CSCD-DBH (Dbh-Cre) | Kindly provided by J. Backs, Heidelberg | |
| Mouse: Ai14 (B6.Cg-Gt(ROSA)26Sortm 14(CAG-tdTomato)Hze/J) | Jackson Laboratories | 007914 |
| Mouse: C3H/HeJ | Jackson Laboratories | 000659 |
| **Software and Algorithms** | | |
| ImageJ v 2.0.0 | Schneider et al., 2012 | https://imagej.nih.gov/ij/ |
| Metamorph v 7.7 | | |
| QuPath v 0.2.0 | Bankhead et al., 2017 | https://qupath.github.io/ |
| Imaris v 9.5 | Bitplane | |
| Seurat v 4.0.2 | Satja lab | |
| Galaxy | -- | -- |
| Prism 8 | GraphPad Software | RRID:SCR 002798 |
| **Other** | | |
| Sequence data, analyses, and resources related to the paper | This paper | |

### 2. Methods

### 2.1 Human tissue samples

Human superior cervical ganglia, pineal glands and parts of left ventricle from heart healthy and donors with heart disease were collected during autopsies. Samples were matched according to age and grouped according to established cardiac disease or controls Use of preserved human specimens was in accordance with the responsible authorities (TUM Ethics Committee). Information regarding cause of death, age, BMI, and heart weight was collected pseudonymously.

### 2.2 Mice

Dbh-Cre mice were a gift from G. Schütz, Heidelberg (13). Dbh-Cre mice were bred with Ai14 (tdTomato^{flox}) mice (from Jackson Laboratory) to generate Dbh-Cre/tdTomato^{flox} mice for imaging experiments. C57BL/6 were purchased from Charles River, C3H/HeJ mice from the Jackson Laboratory, respectively. As many inbred mouse strains including C57BL/6 produce no melatonin, we evaluated pineal gland function on the melatonin-proficient strain C3H/HeJ. For all experiments, 8-10-week-old male mice were used for experiments. All mice were maintained in pathogen-free conditions under a 12 h/12 h light/dark cycle, at a temperature of 21 ± 1°C, a humidity of 40-70% and were fed with autoclaved food. Transverse aortic constriction was performed as previously described (14). Shortly, thoracotomy was performed in order to access the aortic arch which was ligated between the innominate and left carotid arteries. In Sham surgery, the procedure was identical except for the constriction of the aorta. Cardiac function was assessed by echocardiography; aortic constriction was validated by pulse-wave Doppler of the carotid arteries before surgery and before euthanasia. Bilateral removal of SCGs (SCGx) was performed as previously described (5). Blood was obtained from anesthetized mice by puncture of the submandibulary venous plexus and captured in heparinized tubes. Plasma was extracted and prepared according to manufacturer's instructions for photometric measurement of melatonin (ELISA).

Circadian rhythm was assessed by continuous recording of mouse behavior (activity, indirect calorimetry) in Promethion high-definition multiplexed respirometry cages with XYZ beam breaks (Sable Systems) for seven consecutive days. Melatonin was administered as a single intraperitoneal injection (dosage 50 µg). This study complied with all ethical regulations involving experiments with mice, and all experimental procedures performed on mice were approved by the responsible authorities.

### 2.3 Histological stainings

8 µm thick paraffin sections of murine and human superior cervical ganglia were stained with Sirius red and Fast green as previously described (15). Images were taken with a 10x objective using an AxioObserver Z1 (Zeiss, Oberkochen, Germany) motorized scanning stage microscope (130x85; Märzhäuser, Wetzlar, Germany). Metamorph software (Molecular Devices, Dowington, USA; version 7.7) was applied for quantification. Fibrosis was measured as the ratio of Sirius Red to Fast Green signals in each section (MetaMorph). For immunohistochemistry of adrenal glands, 10 µm cryosections were incubated in hydrogen peroxide blocking solution for 10 minutes. After washing and blocking, sections were incubated overnight in antibody directed against tyrosine hydroxylase (ab152, Merck, 1:200 dilution). After washing and incubation in HRP-conjugated secondary anti-rabbit antibody (1:200 dilution), DAB substrate was applied to the sections for 1-3 minutes. Sections were then counterstained with hematoxylin. Analysis was performed with QuPath (version 0.2.0).

### 2.4 Immunofluorescence imaging

Pineal glands from Dbh-Cre/tdTomato mice were harvested and imaged directly. All images were acquired at room temperature using a stereo microscope (M 205 FCA, Leica). Quantification was executed using Imaris (Bitplane, version 9.5) filament tracer tool.

For staining of CD68 cells in superior cervical ganglion, sections were fixated in methanol (10 min -20°C; for human samples) or in 4% PFA (15 min RT, for murine samples). After permeabilization with 1% TritonX and blocking with 2% bovine serum albumin, sections were incubated with primary antibody against CD68 (Human: Thermo, 1:100 dilution; Mouse: Biozol, 1:200 dilution) overnight at 4°C. Nuclei were detected by staining with 4',6-diamidino-2-phenyldole (DAPI). The sections were then incubated with respective secondary antibodies conjugated to Alexa Fluor 568 (Life technologies, 1:200 dilution). Images were acquired using confocal microscopy (Leica TCS SP5 II) and then analyzed with the MetaMorph software.

### 2.5 X-Clarity tissue clearing

For quantification of human pineal gland sympathetic innervation, pineal glands were overnight fixated in 4% PFA at 4°C. After several PBS washes, samples were incubated in Hydrogel-Initiator mixture for 24 hours. Polymerization was executed at 37°C for 3 hours at -87 kPa (Polymerization system, OLS). Samples were cleared in the Tissue X-Clarity System (OLS) actively for 24 hours (37°C, 100 rpm, 1.2 A) and passively for another 24 hours. After clearing, tissue was washed several times with 1X PBS + 0.1% Triton X and incubated for 3 days in primary (anti-TH, Merck, 1:200 dilution) and secondary antibody (AlexaFluor 647 plus #A-32733, Life technologies, 1:200 dilution), respectively. Nuclei were detected by staining with Sytox Green (1:5000 dilution). After staining, tissues were incubated for 5 min in ddH₂O followed by 1-3 hours incubation in X-Clarity Mounting solution (OLS). All images were acquired at room temperature using a stereo microscope (M 205 FCA, Leica). Quantification was executed using Imaris (Bitplane, version 9.5) filament tracer tool.

### 2.6 In Situ Hybridization

For in situ hybridization with immunofluorescence, assays were performed with RNAScope fluorescent multiplex detection reagents (ACDBio) and HybEZ hybridization system (ACDBio) according to the manufacturer's instructions. For colabeling of tissue by in situ hybridization and immunofluorescence, After the last wash, sections were blocked with 1% BSA and 0.1% Triton X-100 for one hour at room temperature and then incubated overnight with primary antibody directed against tyrosine hydroxylase (Merck ab152, 1:200 dilution). Sections were visualized with Alexa Fluor secondary antibodies (ThermoFisher #A-11011, 1:200 dilution) and coverslipped using Prolong Gold Antifade Reagent containing DAPI (ThermoFisher). Images were acquired using confocal microscopy (Leica TCS SP5 II) and then analyzed with the MetaMorph software.

### 2.7 SCG human ultrasound

Ultrasound of human superior cervical ganglia was executed after assessment of cardiac function by echocardiography. Superior cervical ganglia were located as previously described (16). In brief, the carotid arteries were followed cranially. The superior cervical ganglion is located at level of vertebral body C3, anterior to the longus capitis muscle and dorsal to the internal carotid artery. After visualization of the left ganglion, total ganglionic length was assessed. For ultrasound, a Philips EPIQ 7G device was used. The X5-1 transducer was used for echocardiography and the L12-3 linear transducer for SCG ultrasound. This clinical study was in accordance with the Declaration of Helsinki and approved by the local Ethics Committee.

### 2.8 Isolation of primary sympathetic neurons

For the isolation of primary sympathetic neurons, SCGs from at least 15 C57BI/6N male mice at the age between 8-12 weeks were harvested. The ganglia were cleaned and manually minced into small pieces. Afterwards, ganglionic tissue was incubated in artificial cerebrospinal fluid (ACSF) oxygenated by Carbogen with a stabile pH of 7.4 at 37°C for 2 hours. Tissue was then transferred into digestion solution (Collagenase II 2.5 mg/ml with Albumin supplementation 6 mg/ml in ACSF) and incubated for 1 hour at 37°C. After centrifugation and washing in ACSF, the digestion was stopped using Neurobasal-A Medium supplemented with Penicillin/Streptomycin, L-Glutamine and Supplement B27. Cells were then pelleted and mechanically triturated using a glass pipette with an opening diameter of 1 mm and 0.5 mm. The cellular suspension was then passed through a 30 µm filter. After centrifugation, the cell pellet was resuspended in approximately 100-200 µl medium, depending on the pellet size.

### 2.9 Isolation of bone marrow-derived macrophages

For the isolation of bone marrow-derived macrophages, primary bone marrow progenitor cells were harvested from the bones (femur, tibia, humerus, spinal column) of male C57BL/6N mice. Progenitor cells were then differentiated into macrophages for 6 days, using RPMI medium containing recombinant 10 ng/ml murine M-CSF (Peprotech), 1% penicillin/streptomycin and 10% FCS.

### 2.10 Co-culture of sympathetic neurons and bone marrow-derived macrophages

For the co-culture experiment, BMDM precursors were isolated and differentiated to macrophages as mentioned above. On day 6 of differentiation, primary neurons were isolated as described above. ~ 5,000 neurons were seeded on separate wells of a 96-well Ibidi plate precoated with Laminin (0.1 mg/ml). At day 7 of differentiation, macrophages were either stimulated with PBS (control) or LPS (5 ng/ml) overnight. On the following day, LPS-preactivated macrophages were either incubated with PBS (control) or Cobra Venom Factor (CVF; 20 U/ml) for 90 min at 37°C for complement inhibition. Afterwards, macrophages were chemically detached from the Petri dishes using Accutase and seeded around neurons. ~ 10,000 macrophages were added per well. Co-culture medium was a mixture of neuronal and macrophage medium. Furthermore, part of the co-culture medium was supplemented with 1 µM Nicotine for artificial neuronal stimulation. 4 wells were cultivated per condition. One well per condition was transferred into the EVOS M7000 for live cell imaging (timelapse). Automatic focusing (on small structures) was applied. Every 30 minutes a 3x3 tile scan was acquired per well for 48 hours.

After 48 hours of co-culture (at 37°C and 5% CO₂), cells were fixated using 4% Paraformaldehyde. After several wash steps with PBS, cells were permeabilized using 0.2% Triton X-100 and blocked with 2% bovine serum albumin (BSA). Primary antibodies directed against β-Tubulin III (Biolegend #AA-10, dilution 1:250) and CD68 (Biorad #MCA1957, dilution 1:200) were added to the wells and incubated for 1 hour at 37°C. Wells were then washed with PBS and incubated with secondary antibodies (AlexaFluor goat anti-mouse 488 #A-11029, AlexaFluor goat anti-rat 594 #A-11007, dilution 1:200) at 37°C for 1.5 hours. DAPIwas used for nuclei detection. After washing, wells were immersed in 50% glycerol for imaging. For quantification, two tilescans (2x2) per well were acquired using a 63x objective of a confocal microscope (Leica TCS SP5 II) and quantified for neuronal cell number and axonal dimensions using Imaris (Bitplane, version 9.5). Representative images of macrophage accumulation at the axon initiation segment were acquired using a 40x objective of the EVOS M7000.

### 2.11 Single cell RNA sequencing

For single cell RNA sequencing, neurons were isolated as mentioned above (see isolation of primary sympathetic neurons). Cells were isolated from wild type mice 5 and 18 days after TAC as well as after Sham surgery. Approximately 10,000 cells were loaded into a single channel of the Chromium system (10X Genomics) for each of the samples, and the libraries were prepared according to manufacturer's instructions. Illumina sequencing was then carried out using HiSeq4000.

Raw sequencing data was processed with Cellranger (v 5.0.0; 10X Genomics) and Seurat (v 4.0) for downstream analysis. Sequencing reads were aligned to mouse reference genome 10 (mm10) (Genome Reference Consortium). Cells with < 500 genes or > 4000 genes or mitochondrial genes greater than 15-25% were filtered out. Additionally, in the Sham dataset cell debris was removed. Expression data was log-normalized, and highly variable genes were detected for linear dimensional reduction using principal component analysis (PCA). Altogether, the filtered data contained 11,608 cells. The objects were combined into a single object, using the RunFastMNN algorithm that scales the values of the two datasets relative to each other and align the cells in the same manner. Dimensionality reduction and unsupervised clustering of cells, as implemented in the Seurat package, was then carried out to identify distinct cell populations within the large data set. This approach allowed to carry out a single integrated analysis on all cells. To reveal heterogeneity within the major cell types, we carried out cell clustering at a low resolution of 0.1. FindMarkers tool within the Seurat package was then used to identify individual marker genes for each cluster. Ligand receptor analysis was performed using the iTALK R package (17). Hereby, intercellular interactions as well as disease-dependent changes in intercellular communication were assessed. For the analysis, a database containing all known ligand receptor pairs (approximately 2200) from the recently published algorithm "cellphone DB" (18) was used. For analysis of neuroimmune interactions, a subset containing neuronal (sympathetic_other and sympathetic_pineal) and immune cells (macrophages, monocytes, T_cells, DC-like_cells) was created. The different immune cell types were identified by clustering at a higher resolution of 0.3. For the analysis of TAC-induced interaction changes, ligand-receptor pairs with a mean expression of the ligand greater than 2 were considered. The networks were plotted using iTALK and igraph R packages.

### 2.12 Single nuclear RNA sequencing

Nuclei were isolated as previously described by Mathys et al (19). Cells were isolated from wild type mice 5 after TAC and Sham surgery. Approximately 10,000 nuclei were loaded into a single channel of the Chromium system (10X Genomics) for each of the samples, and the libraries were prepared according to manufacturer's instructions. Illumina sequencing was then carried out using HiSeq4000.

First, Cellranger (v 5.0.0, 10X Genomics) *mkref* with the parameter --include introns was used to build a custom "pre-mRNA" annotation. Raw sequencing data was then processed with Cellranger (v 5.0.0, 10X Genomics) *count* using default parameters and mouse reference genome 10 (mm10) (Genome Reference Consortium). Seurat (v 4.0) was used for downstream analysis as follows. Cells with < 400 genes or > 4000 genes or mitochondrial genes greater than 1.0-1.5% were filtered out. Expression data was log-normalized, cell cycle scoring as well as SCtransformation was applied. Altogether, the filtered data contained 9,172 cells. The objects were combined into a single object. Dimensionality reduction and unsupervised clustering of nuclei, as implemented in the Seurat package, was then carried out to identify distinct cell populations within the large data set. After integration, zero-preserving imputation using low-rank approximation (ALRA (20)) was applied. Dimensionality reduction and unsupervised clustering of nuclei, as implemented in the Seurat package, was then carried out to identify distinct cell populations within the large data set. This approach allowed to carry out a single integrated analysis on all nuclei. To reveal heterogeneity within the major cell types, we carried out cell clustering at a resolution of 0.3. FindMarkers tool within the Seurat package was then used to identify individual marker genes for each cluster.

### 2.13 RNA Seq and genetic deconvolution

RNeasy midi-prep kit was used for the extraction of RNA from human SCGs according to manufacturer's instructions, RNeasy mini-prep kit was used for the extraction of RNA from murine SCGs, respectively. After quality control by RIN value (for human SCGs cutoff of RIN ≥ 5.0), RNA libraries were prepared using TruSeq Stranded mRNA kit (Illumina) and sequenced using 75 base-pair single end reads on a NextSeq 500 instrument (Illumina; for hSCG libraries) or using 100 bp paired-end reads on Illumina HiSeq 4000 (for mSCG and murine pineal gland libraries).

Reads were aligned to human GRCh38 (version 34, Ensembl 100) and mouse GRCm38 (version M21, Ensembl 96) using STAR. Gene abundance files were calculated using StringTie, differential gene expression was assessed using the DESeq2. In total, 6 samples from human left SCG matched the pre-defined quality criteria (enriched Th-expression, i.e., TPM ≥ 50).

RNA from pineal glands was extracted using Trizol reagent (Thermo Fisher Scientific) according to the manufacture's protocol. RNA sequencing was performed as described above. Reads were aligned to mouse GRCm38 (version M23, Ensembl 98) using STAR. Gene abundance files were calculated using StringTie, differential gene expression was assessed using the DESeq2 tool. Human and mouse bulk RNA deep seq data set was then computed using the online tool CIBERSORT (http://cibersort.stanford.edu/) (20). For genetic deconvolution of human data, we transformed the signature matrix from murine SCGs to the corresponding human genes using the biomaRt R package. For genetic deconvolution of genes expressed by murine pineal glands, we used the recently published single cell dataset from rat pineal gland (21) and analyzed it using Seurat suite version 4.0 on R Studio (version 1.2.5033) platform. A signature matrix containing cell type-specific marker genes for the main cell populations - pinealocytes, glial cells, microglia and leptomeningeal/endothelial cells - was created. For genetic deconvolution of murine data, we transformed the signature matrix from rat pineal glands to the corresponding mouse genes using the biomaRt R package. Radar charts were plotted with individual scale bars using the fmsb package.

### 2.14 Electron microscopy

For electron microscopy, mice were perfused for 20 min in 4% PFA, 2.5% glutaraldehyde (EM-grade, Science Services), 2 mM calcium chloride in 0.1 M sodium cacodylate buffer (pH 7.4) (Sigma Aldrich). The tissue was fixed for another 24-48 h at 4°C in perfusion buffer and stored in 0.1 M sodium cacodylate buffer. Afterwards, we applied a standard rOTO en bloc staining protocol (22) including post-fixation in 2% osmium tetroxide (EMS), 1.5% potassium ferricyanide (Sigma) in 0.1 M sodium cacodylate (Science Services) buffer (pH 7.4). Staining was enhanced by reaction with 1% thiocarbohydrazide (Sigma) for 45 min at 40°C. The tissue was washed in water and incubated in 2% aqueous osmium tetroxide, washed, and further contrasted by overnight incubation in 1% aqueous uranyl acetate at 4°C and 2h at 50°C. Samples were dehydrated in an ascending ethanol series and infiltrated with LX112 (LADD). For the volume analysis, the block was trimmed, serial sections taken (Powertome, RMC) with a 35° ultra maxi diamond knife (Diatome) at a nominal cutting thickness of 100 nm and collected on freshly plasma-treated (custom-built, based on Pelco easiGlow, adopted from M. Terasaki, U. Connecticut, CT), carbon nanotube (CNT) tape (Science Services). CNT tape stripes were assembled onto adhesive carbon tape (Science Services) attached to 4-inch silicon wafers (Siegert Wafer) and grounded by adhesive carbon tape strips (Science Services). EM micrographs were acquired on a Crossbeam Gemini 340 SEM (Zeiss) with a four-quadrant backscatter detector at 8 kV. In ATLAS5 Array Tomography (Fibics), wafer overview images were generated (1333 nm/pixel). We acquired low resolution (200 x 200 x 100 nm) volumes of ganglia serial longitudinal sections and a high resolution (10 x 10 x 100 nm) data set (990 x 1060 x 22 µm). Serial section data were aligned by a sequence of automated and manual processing steps in Fiji TrakEM2 (23). VAST software was used for manual segmentation (24) and Blender for rendering (25) of macrophages and neurons.

### 2.15 Statistical analysis

Data were presented as mean ± SEM. Statistical analysis was performed using GraphPad Prism software package (version 8). Data distribution was assessed by D'Agostino-Pearson omnibus test, Shapiro-Wilk test, and Kolmogorov-Smirnov test for normality. Differences between the two means were tested using two-tailed unpaired Student's t-test for Gaussian distributed data. Analysis of variance followed by Bonferroni test (normal distributed data set) or Kruskal-Wallis test (non-normal distributed data set) were used as appropriate and indicated in the figure legend. P<0.05 was considered statistically significant.

### 3. Results

### 3.1 Cardiac disease causes sympathetic denervation and dysfunction of the pineal gland

Given the key role of the pineal gland in circadian rhythms and its peripheral sympathetic regulation (26) and the high prevalence of both circadian (12) and sympathetic disruptions (27) in chronic cardiac disease, we hypothesized that in such pathological settings the neuronal control of pineal gland function might be impaired. We assessed pineal gland sympathetic innervation in humans with cardiac disease compared to heart healthy controls. We collected post-mortem pineal glands from 7 heart disease patients and 9 heart healthy controls, performed tissue clearing and stained for the sympathetic marker enzyme, tyrosine hydroxylase. In pineal gland tissue from the patients with cardiac disease background, we observed a significant reduction of axonal area and length (Fig. 1a-b).

We reasoned that this loss of innervation of the pineal gland could provide an explanation for the lower melatonin levels observed in humans with cardiac disease (28) and therefore sought to investigate the underlying cellular mechanism in an animal model of cardiac disease. We surveyed pineal gland function in mice subjected to transverse aortic constriction (TAC) to exert left ventricular pressure overload, a condition that leads to pathologic cardiac hypertrophy and failure (29) (Fig. 1c-e, Fig. S1a). Plasma melatonin concentrations in these TAC-treated mice were reduced, relative to controls, 4 weeks after the respective intervention (Fig. 1d).

We then asked if this change in pineal gland function causes alterations of a comprehensive standard set of parameters for diurnal rhythmicity including respirometry to survey metabolic rates, nutrient uptake, body mass as well as activity (determined by infrared arrays). We found a marked disruption of diurnal rhythmicity with a reduced amplitude in TAC mice compared to controls. Importantly, this relative loss of diurnal rhythmicity was not associated with changes in total energy expenditure or activity, indicating that the shift in cycling is a specific effect rather than being related to unspecific sickness of TAC mice (Fig. 1e-f).

Mice allow the genetic labeling of sympathetic neurons with superior signal-to-noise ratio axon imaging (30) and we sought to generate mice with fluorescently labelled sympathetic axons by cross breeding of dopamine-p-hydroxylase (Dbh)-Cre mice with tdTomato^{flox} mice (Fig. S1b). Application of cardiac pressure overload for 4 weeks recapitulated the hallmarks of pineal gland pathology observed in humans with chronic cardiac disease, namely a significantly lower sympathetic axonal area and length compared to sham-operated animals (Fig. 1g-h). Pineal gland cellular composition, however, remained unchanged (Fig. S1c), prompting us to analyze next the SCG from which the pineal gland-innervating axons originate.

### 3.2 Chronic cardiac disease causes fibrotic scarring and SCG hypertrophy in mice and humans

Morphometry and histopathology of SCGs derived from mice subjected to TAC revealed significant hypertrophy, with significantly higher ganglionic volumes (Fig. 2a-b). Staining of histopathological sections with Sirius Red/Fast Green further detected extensive fibrotic scarring of SCGs in TAC mice compared to sham-operated animals suggesting massive, possibly irreversible organ damage (Fig. 2a-b).

We then asked whether these findings can be translated to chronic cardiac disease in humans and prospectively obtained a total of 38 SCG specimens from 19 individuals upon autopsy (see Fig. S2a for patient characteristics). As in mice with cardiac disease, SCGs from heart disease patients were significantly enlarged and exhibited increased interstitial scarring, with the stained matrix volume amounting for approx. 70% of total intra-ganglionic volume (Fig. 2c-d). Interestingly, the extent of ganglionic fibrosis significantly correlated with the extent of myocardial remodeling (Fig. S2b).

The remarkable degree of SCG hypertrophy led us to speculate that the size of the SCG might serve as an imaging biomarker for heart failure. In a clinical study, we therefore prospectively evaluated SCG dimensions by quantitative ultrasound imaging in heart failure patients and healthy controls (Fig. 2e-f, Fig. S3a-b). Evaluation with current clinical ultrasound equipment revealed a significant increase of SCG dimensions in patients with heart failure compared to heart healthy controls (defined by ejection fraction, Fig. 2g), with eight out of nine patients displaying an approx. 150% higher SCG length. In line with a functional interdependence, a significant correlation between SCG size and ejection fraction was observed (Fig. S3c).

### 3.3 Heart disease triggers macrophage infiltration and loss of pineal gland-innervating neurons in SCGs.

To dissect and quantitatively assess the cellular composition that underlies the histomorphologic alterations of the SCG, we performed single cell (scRNA seq) and single nuclei (snRNA seq) RNA sequencing. Ganglia were isolated from control mice and mice that had been subjected to TAC (Fig. 3a). In total, 20,780 cells passed the quality control and were used as input for the computational analysis. The cellular compendium comprised five major cell types, namely sympathetic neurons, glial cells, fibroblasts, endothelial cells, and immune cells (Fig. 3a). Single-nuclei sequencing furthermore identified two distinct cell clusters among the sympathetic neurons, one of which selectively expressed melatonin receptor 1A (*Mtnr1a*). *Mtnr1a* has been characterized as the essential guidance cue in pineal gland-innervating neurons that promotes sympathetic innervation of the melatonin-secreting pineal gland during development (31) (Fig. 3a, Fig. S4).

Our sequencing analyses showed that pineal gland-innervating neurons share tyrosine hydroxylase (*Th*)*,* dopamine-p-hydroxylase (*Dbh*)*,* Neuropeptide Y (*Npy*), peripherin (*Prph*)*,* and Synapsin II (*Syn2*) as typical markers for sympathetic neurons (in line with other reports of state-of-the-art cell inventories of sympathetic ganglia from Zeisel (32) and Furlan (6)). In addition, pineal gland-innervating neurons were characterized by expression of small nucleolar RNA host gene 11 (*Snhg11*)*,* calcium voltage-gated channel subunit alpha1 A (*Cacna1a*), Ankyrin 2 (*Ank2*)*,* Hand2os1, Semaphorin 6D (*Sema6d*)*,* EPH Receptor A5 (*Epha5*)*,* neuronal cell adhesion molecule (*Nrcam*)*,* and synaptosome associated protein 91 (*Snap91*)*.* These data identify at least two distinct neuron populations in the mouse SCG, with one representing the pineal gland-innervating fraction known to control melatonin secretion in mammals. Next to glial cells, a remarkably high number of immune cells was detected within the SCG, the majority of which are macrophages (single cell dataset: 5-8% cells positive for macrophage marker, data not shown).

Differential expression analysis of the single cell data then permitted us to create a signature matrix of marker genes specific for the main cell populations in the SCG. This matrix was then used as a basis for genetic deconvolution (20) of deep RNA sequencing data of murine and human ganglia (see Fig. S5a for an illustrated workflow).

Ganglia from mice that had been subjected to TAC displayed a marked increase of the macrophage cell fraction, as well as a significant reduction of sympathetic pineal gland-innervating neurons ('sympathetic pineal'; Fig. 3b) at day 18 after the procedure, and thus well before decompensated heart failure was fully established (day 28 day) (Fig. S5b).

In line with previous observations, sympathetic hyperactivity indicated by hypertrophy of the adrenal medulla was detected in mice subjected to TAC (Fig. S5c). To independently validate these findings, tissue cryosections of SCGs obtained from mice subjected to TAC or control mice were stained for the macrophage marker CD68 (antibody, Fig. 3c) and probed for mRNA expression of the sympathetic pineal gland-innervating neuronal cell marker gene, *Mtnr1a* (*in situ* hybridization, Fig. 3d). Quantitative analysis confirmed a significant increase of CD68⁺ macrophages and a significant loss of *Mtnr1a*⁺ neurons (Fig. 3c-d). The loss of sympathetic pineal gland-innervating neurons was already significant 18 days after TAC and rapidly progressed towards an almost entire loss of *Mtnr1a*⁺ neurons (Fig. 3d). Transcriptome analysis of human SCG autopsy samples from patients with established cardiac disease recapitulated these findings, namely a significant macrophage infiltration (albeit to a lesser extent at this late stage of cardiac disease) and the loss of sympathetic pineal gland-innervating neurons (Fig. 3b). Immunofluorescent detection of intraganglionic macrophages in cryosections of these samples confirmed this finding (Fig. 3e).

### 3.4 Loss of pineal gland-innervating neurons is mediated by activated macrophages

The observed infiltration of the SCG by macrophages and the concomitant loss of sympathetic pineal gland-innervating neurons prompted us to speculate that these findings may be related. We therefore aimed to localize macrophages and their spatial orientation with regard to neurons by volume electron microscopy (3D-EM) in SCGs obtained at early stages of cardiac disease, i. e., before the loss of sympathetic pineal gland-innervating neurons. 3D-EM of ganglia from day 5 after TAC revealed two striking observations at this early disease state, namely electron dense alterations at the axon initiation segment (AIS) of neurons and closely co-localizing macrophages (Fig, 4a, Fig. S6). Previous work has established such changes in electron density as a possible sign of local reactive oxygen species-related damage (33) that resulted e.g. from a local cytotoxic immune attack. Indeed, the macrophages found nearby the electron-dense AIS showed such an activated phenotype, as indicated by a high abundance of lysosomes (34).

Next, we investigated potential macrophage-neuron interactions in a defined in vitro setting by co-culturing macrophages and sympathetic neurons. To model the respective disease-associated cell states, we pre-activated macrophages with lipopolysaccharide (LPS) to induce a pro-inflammatory (M1-like) state, while cardiac disease-associated over-activation of sympathetic neurons was mimicked by chronic treatment with nicotine (1 µM for 48 h). In this co-culture system, migration of activated macrophages towards the AIS was apparent, a behavior that was dependent on prior nicotine treatment of neurons (Fig. 4b-c, supplementary Video 1-2). To validate this finding of crucial macrophage-neuron interaction, we next mapped intercellular communication in SCGs by transcriptome profiling (18). At early stages of cardiac disease (d5 after TAC), this analysis revealed the most pronounced alterations to occur in the communication network between macrophages and sympathetic pineal gland-innervating neurons (Fig. 4d).

To further evaluate if macrophages promote cell death of peripheral sympathetic neurons, we surveyed cell survival in a defined co-culture setting of macrophages and sympathetic neurons (Figure 4f-g). Co-culture with pro-inflammatory (M1-like), but not with control macrophages inhibited neurite outgrowth and induced cell loss of nicotine-stimulated, but not of quiescent neurons (Fig. 4e-f and Fig. S7a-b). Treatment with Cobra venom factor (CVF), a broad spectrum complement inhibitor that inhibits macrophage activation (35), effectively prevented both neuronal cell loss and rescued neurite outgrowth in this setting (Fig. 4f). Together, these data identify a central role for activated, pro-inflammatory macrophages in cell death of sympathetic neurons. As a further approach to evaluate our results, we mimicked neuronal loss in a mouse model of pineal gland denervation by bilateral removal of the SCGs (SCGx) and surveyed diurnal rhythm (Fig. 4g-h). SCGx resulted in an almost complete disruption of diurnal rhythm (Fig. 4h). Diurnal rhythmicity, however, could be completely restored by supplementation of melatonin (Fig. 4h).

### 3.5 Inhibition of macrophages by local injection of clodronate

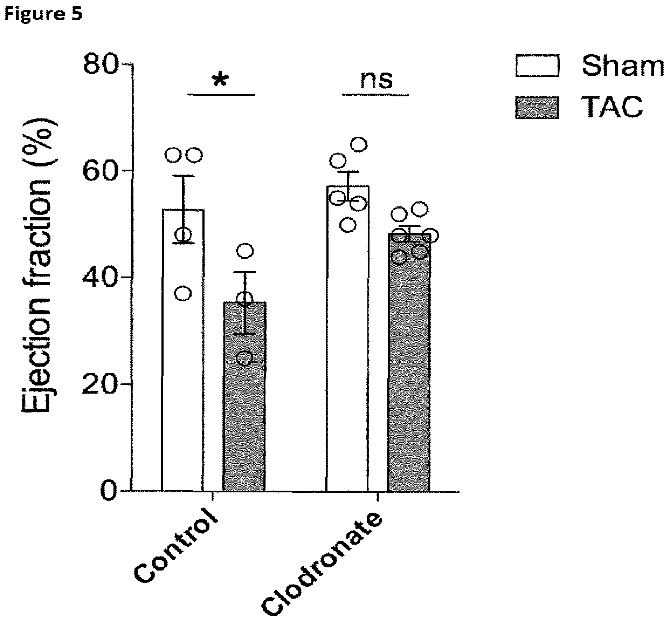

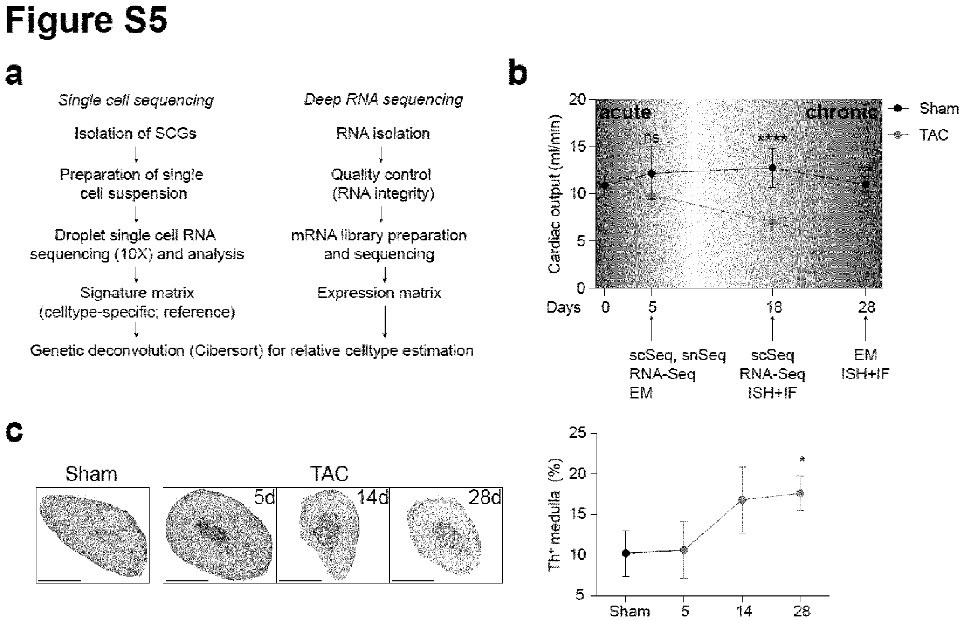

To investigate if local macrophage inhibition decreases sympathetic dysfunction and/or cell death and thereby preserves cardiac and pineal gland function, we applied the well-established macrophage inhibitor clodronate, a bisphosphonate compound, to mice subjected to TAC-induced heart failure. 4 days after TAC and thereafter at weekly intervals, clodronate liposomes were microinjected bilaterally into SCGs. Echocardiography demonstrated that application of clodronate liposomes attenuated TAC-induced cardiac dysfunction (Fig. 5). These data support our hypothesis that interference with ganglionic inflammation may constitute a viable therapeutic strategy in cardiac disease.

### 3.6 Calcification of the pineal gland with decreased melatonin production is associated with sympathetic denervation of the pineal gland

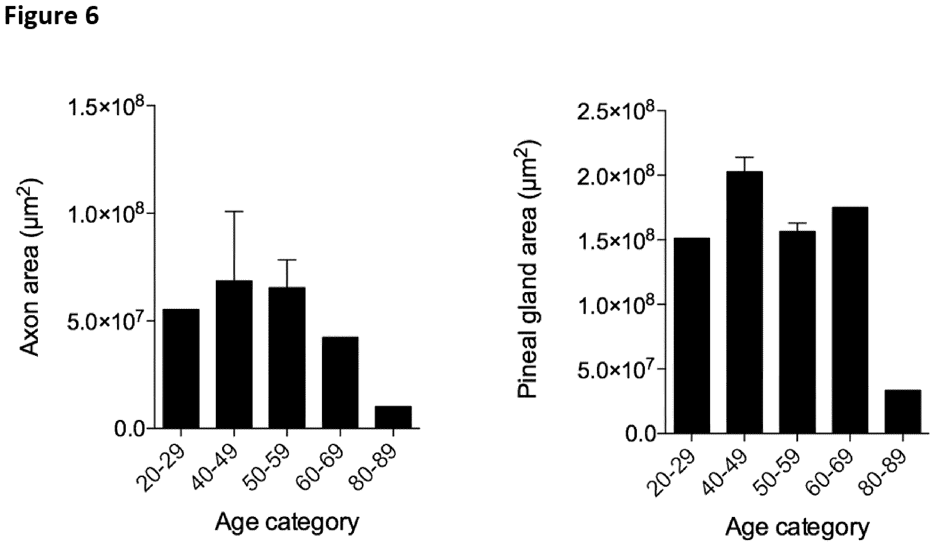

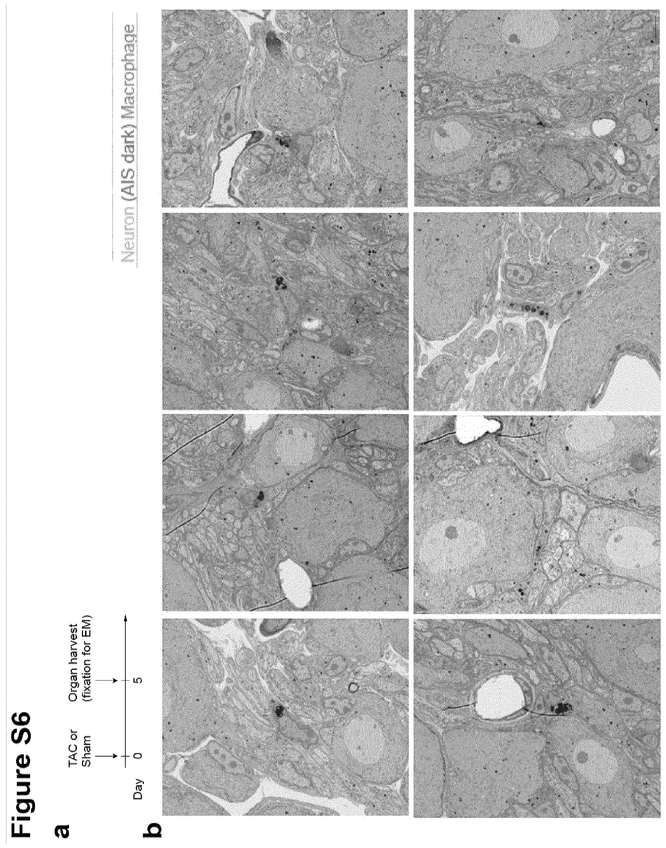

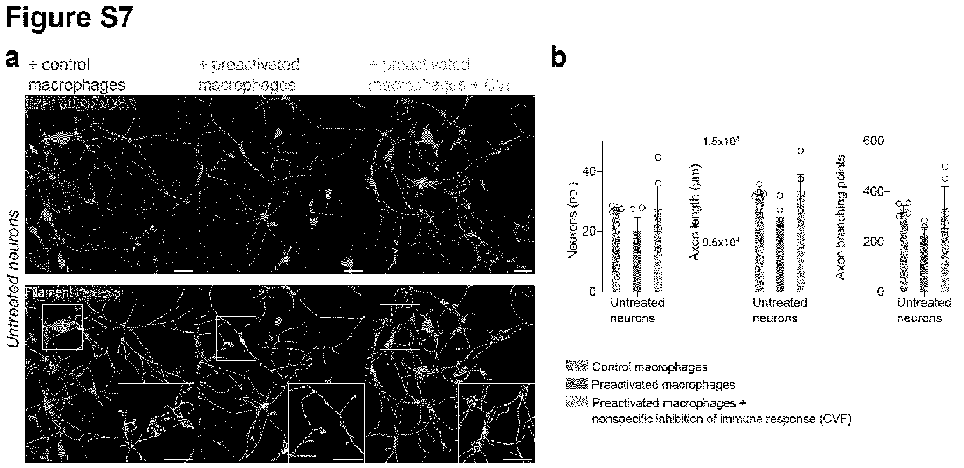

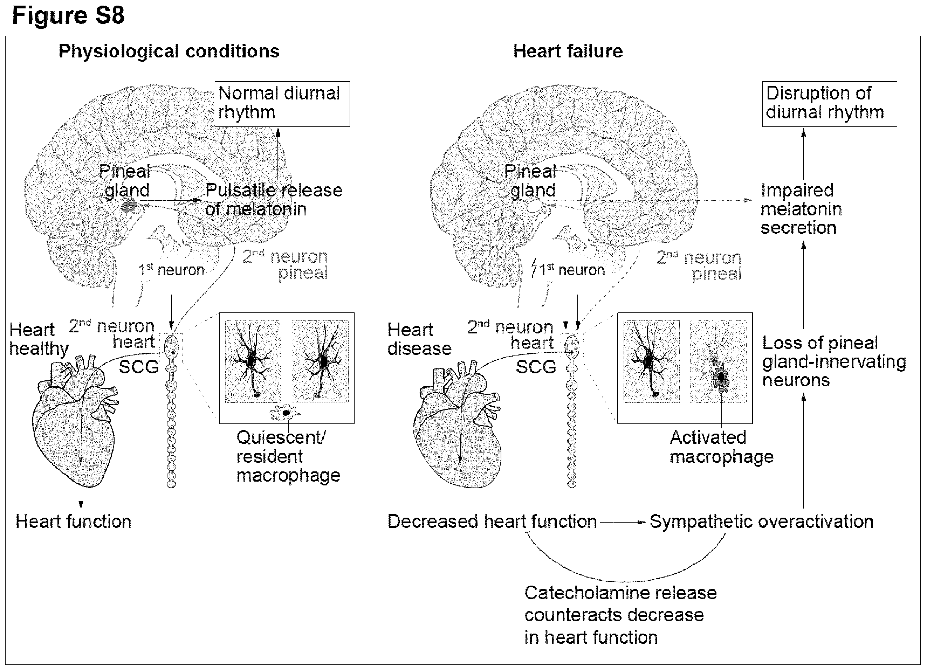

It is well established that pineal gland calcification is accompanied by impaired function, i.e., melatonin production and both pineal gland calcification and melatonin deficiency correlate with increasing age (36, 37). Here, we asked, whether this observation may be related to a potential decrease of sympathetic innervation of the pineal gland with increasing age. Indeed, quantitative analysis of pineal gland size as well as sympathetic axon area in human subjects post-mortem revealed an age-dependent decrease in both parameters (Fig. 6). These data suggest that therapeutic intervention with inflammation of the SCG may prove as an effective means to prevent or halt the progression of age-dependent pineal gland denervation, atrophy, dysfunctional melatonin synthesis and pineal gland calcification.

### 4. Discussion

Disruption of the sleep-wake pattern and melatonin secretion is an established consequence of cardiac disease, yet its mechanistic basis has remained elusive.

We report that cardiovascular disease triggers the pathological alteration of peripheral. neuronal circuit resulting in sympathetic denervation of the pineal gland. We traced the cellular basis of defective pineal gland neuronal control back to selective neuron death in the upper neck ganglion (SCG) which co-innervates both the pineal gland and the heart. Our findings suggest a pathomechanism, where chronic overactivation of the sympathetic nervous system, a hallmark of heart disease (27), triggers detrimental ganglionic adaptations and promotes neuronal susceptibility to pathological inflammatory responses. The respective experiments on co-cultured sympathetic neurons and macrophages further suggest a rationale, how cellular homeostasis is preserved under physiological conditions and periods of sympathetic activation. Neither isolated inflammatory polarization of the macrophages, nor chronic activation of sympathetic neurons were sufficient to induce neuronal cell loss, but the latter depended on their simultaneous occurrence, a condition typically met in the SCG during heart failure.

Neuronal hypertrophy as a sign of local and systemic sympathetic overactivation has been reported in the stellate ganglion (38) and in the adrenal medulla (39), which we were able to confirm (Fig. S5c). The SCG, which also innervates the heart, has not been characterized in this regard. A principal difference between the SCG and other sympathetic ganglia is the pineal gland-innervating neuronal subpopulation that proved highly susceptible to cellular stress in disease conditions. The fundamentally different cellular adaptation processes that occur within the SCG and other sympathetic ganglia are also evidenced by the lack of fibrotic tissue remodeling in the stellate ganglion (38) and adrenal gland (40) in contrast to the irreversible fibrosis that we identified in the SCG.

We foresee three objectives of immediate clinical relevance for this study. First, the identification of defective melatonin secretion underlying sleep abnormalities in patients with cardiac disease suggests that the low melatonin levels reported in these patients (28) must be interpreted to causally contribute to their sleep disturbance. Thus, the further systematic clinical investigation of melatonin administration as a potential treatment regime appears warranted (41).

Second, fibrotic remodeling of tissues and organs is typically regarded as a sign of irreversible tissue damage (42) and the above suggested supplementation of melatonin will necessarily remain symptomatic. If detected at early stages of ganglionic damage, the application of preventive, anti-inflammatory interventions may save patients from the putatively irreversible damage of their autonomic ganglion structure and function. While not directly tested in the present study, this would be in line with previous studies in CNS disease, suggesting that therapeutic interference with microglia (central nervous system analogs to macrophages) activation may promote neuronal survival (33, 43-47).

Third, our findings on the pronounced hypertrophy of the SCG provide a perspective to establish a simple and robust biomarker to identify those cardiac patients that are at risk for pineal denervation and sleep disorders and are hence candidates for the above mentioned therapeutic and preventive interventions. Agonists or antagonists of these biomarkers may also serve as therapeutic agents in preventing and/or treating pineal denervation.

Our study may also proof relevant for non-cardiovascular disease. It suggests a novel paradigm that permits the spread of locally confined organ disease to anatomically distant organs and that is based on the integration of organ-specific neuronal subpopulations in peripheral ganglia (Fig. S8).

### References

1. R. S. Edgar, E. W. Green, Y. Zhao, G. Van Ooijen, M. Olmedo, X. Qin, Y. Xu, M. Pan, U. K. Valekunja, K. A. Feeney, E. S. Maywood, M. H. Hastings, N. S. Baliga, M. Merrow, A. J. Millar, C. H. Johnson, C. P. Kyriacou, J. S. O'Neill, A. B. Reddy, Peroxiredoxins are conserved markers of circadian rhythms. Nature. 485, 459-464 (2012).
2. J. C. Dunlap, Molecular bases for circadian clocks. Cell. 96, 271-290 (1999).
3. H. Lynch, R. Wurtman, M. Moskowitz, M. Archer, M. Ho, Daily rhythm in human urinary melatonin. Science (80-.). 187, 169-171 (1975).
4. A. Végh, S. Duim, A. Smits, R. Poelmann, A. ten Harkel, M. DeRuiter, M. Goumans, M. Jongbloed, Part and parcel of the cardiac autonomic nerve system: Unravelling its cellular building blocks during development. J. Cardiovasc. Dev. Dis. 3, 28 (2016).
5. K. a. Ziegler, A. Ahles, T. Wille, J. Kerler, D. Ramanujam, S. Engelhardt, Local sympathetic denervation attenuates myocardial inflammation and improves cardiac function after myocardial infarction in mice. Cardiovasc. Res. 114, 291-299 (2018).
6. A. Furlan, G. La Manno, M. Lübke, M. Haring, H. Abdo, H. Hochgerner, J. Kupari, D. Usoskin, M. S. Airaksinen, G. Oliver, S. Linnarsson, P. Ernfors, Visceral motor neuron diversity delineates a cellular basis for nipple- and piloerection muscle control. Nat. Neurosci. 19, 1331-1340 (2016).
7. J. Hadaya, J. L. Ardell, Autonomic Modulation for Cardiovascular Disease. Front. Physiol. 11 (2020), doi:10.3389/fphys.2020.617459.
8. T. Eschenhagen, β-adrenergic signaling in heart failure-adapt or die. Nat. Med. 14, 485-487 (2008).
9. S. T. Anderson, G. A. FitzGerald, Sexual dimorphism in body clocks. Science (80-. ). 369, 1164-1165 (2020).
10. I. Garcia-Lunar, V. Fuster, B. Ibanez, Good night, sleep tight. Eur. Heart J. 42, 2100-2102 (2021).
11. B. M. Kuehn, The Heart's Circadian Rhythms Point to Potential Treatment Strategies. Circulation. 134, 1907-1908 (2016).
12. S. S. Thosar, M. P. Butler, S. A. Shea, Role of the circadian system in cardiovascular disease. J. Clin. Invest. 128, 2157-2167 (2018).
13. R. Parlato, C. Otto, Y. Begus, S. Stotz, G. Schütz, Specific ablation of the transcription factor CREB in sympathetic neurons surprisingly protects against developmentally regulated apoptosis. Development. 134, 1663-1670 (2007).
14. H. A. Rockman, R. S. Ross, A. N. Harris, K. U. Knowlton, M. E. Steinhelper, L. J. Field, J. Ross, K. R. Chien, Segregation of atrial-specific and inducible expression of an atrial natriuretic factor transgene in an in vivo murine model of cardiac hypertrophy. Proc. Natl. Acad. Sci. 88, 9907-9907 (1991).
15. H. Nakajima, H. O. Nakajima, O. Salcher, A. S. Dittie, K. Dembowsky, S. Jing, L. J. Field, Atrial but Not Ventricular Fibrosis in Mice Expressing a Mutant Transforming Growth Factor- 1 Transgene in the Heart. Circ. Res. 86, 571-579 (2000).
16. A. Siegenthaler, M. Haug, U. Eichenberger, M. R. Suter, B. Moriggl, Block of the Superior Cervical Ganglion, Description of a Novel Ultrasound-Guided Technique in Human Cadavers. Pain Med. (United States). 14, 646-649 (2013).
17. Y. Wang, R. Wang, S. Zhang, S. Song, C. Jiang, G. Han, M. Wang, J. Ajani, A. Futreal, L. Wang, iTALK: an R Package to Characterize and Illustrate Intercellular Communication (2019), doi:10.1101/507871.
18. M. Efremova, M. Vento-Tormo, S. A. Teichmann, R. Vento-Tormo, CellPhoneDB: inferring cell-cell communication from combined expression of multi-subunit ligand-receptor complexes. Nat. Protoc. 15, 1484-1506 (2020).
19. H. Mathys, J. Davila-Velderrain, Z. Peng, F. Gao, S. Mohammadi, J. Z. Young, M. Menon, L. He, F. Abdurrob, X. Jiang, A. J. Martorell, R. M. Ransohoff, B. P. Hafler, D. A. Bennett, M. Kellis, L. H. Tsai, Single-cell transcriptomic analysis of Alzheimer's disease. Nature. 570, 332-337 (2019).
20. A. M. Newman, C. L. Liu, M. R. Green, A. J. Gentles, W. Feng, Y. Xu, C. D. Hoang, M. Diehn, A. A. Alizadeh, Robust enumeration of cell subsets from tissue expression profiles. Nat. Methods. 12, 453-457 (2015).
21. J. C. Mays, M. C. Kelly, S. L. Coon, L. Holtzclaw, M. F. Rath, M. W. Kelley, D. C. Klein, Single-cell RNA sequencing of the mammalian pineal gland identifies two pinealocyte subtypes and cell type-specific daily patterns of gene expression. PLoS One. 13, 1-22 (2018).
22. G. Kislinger, H. Gnägi, M. Kerschensteiner, M. Simons, T. Misgeld, M. Schifferer, ATUM-FIB microscopy for targeting and multiscale imaging of rare events in mouse cortex. STAR Protoc. 1, 100232 (2020).
23. J. Schindelin, I. Arganda-Carreras, E. Frise, V. Kaynig, M. Longair, T. Pietzsch, S. Preibisch, C. Rueden, S. Saalfeld, B. Schmid, J. Y. Tinevez, D. J. White, V. Hartenstein, K. Eliceiri, P. Tomancak, A. Cardona, Fiji: An open-source platform for biological-image analysis. Nat. Methods. 9, 676-682 (2012).
24. D. R. Berger, H. S. Seung, J. W. Lichtman, VAST (Volume Annotation and Segmentation Tool): Efficient manual and semi-automatic labeling of large 3D image stacks. Front. Neural Circuits. 12 (2018), doi:10.3389/fncir.2018.00088.
25. Allan Brito, Blender Quick Start Guide 3D Modeling, Animation, and Render with Eevee in Blender 2.8 (Packt Publishing, 2018).
26. J. Axelrod, The pineal gland: A neurochemical transducer. Science (80-. ). 184, 1341-1348 (1974).
27. E. N. Bardsley, D. J. Paterson, Neurocardiac regulation: from cardiac mechanisms to novel therapeutic approaches. J. Physiol. 598, 2957-2976 (2020).
28. P. Brugger, W. Marktl, M. Herold, Impaired nocturnal secretion of melatonin in coronary heart disease. Lancet. 345, 1408 (1995).
29. H. A. Rockman, R. S. Ross, A. N. Harris, K. U. Knowlton, M. E. Steinhelpert, L. J. Fieldt, J. Ross, K. R. Chien, M. E. Steinhelper, L. J. Field, Segregation of atrial-specific and inducible expression of an atrial natriuretic factor transgene in an in vivo murine model of cardiac hypertrophy. Proc. Natl. Acad. Sci. U. S. A. 88, 8277-81 (1991).
30. R. M. Pirzgalska, E. Seixas, J. S. Seidman, V. M. Link, N. M. Sanchez, I. Mahú, R. Mendes, V. Gres, N. Kubasova, I. Morris, B. a. Arús, C. M. Larabee, M. Vasques, F. Tortosa, A. L. Sousa, S. Anandan, E. Tranfield, M. K. Hahn, M. Iannacone, N. J. Spann, C. K. Glass, A. I. Domingos, Sympathetic neuron-associated macrophages contribute to obesity by importing and metabolizing norepinephrine. Nat. Med. 23, 1309-1318 (2017).
31. I. B. Black, C. Mytilineou, Trans-synaptic regulation of the development of end organ innervation by sympathetic neurons. Brain Res. 101, 503-521 (1976).
32. A. Zeisel, H. Hochgerner, P. Lönnerberg, A. Johnsson, F. Memic, J. van der Zwan, M. Haring, E. Braun, L. E. Borm, G. La Manno, S. Codeluppi, A. Furlan, K. Lee, N. Skene, K. D. Harris, J. Hjerling-Leffler, E. Arenas, P. Ernfors, U. Marklund, S. Linnarsson, Molecular Architecture of the Mouse Nervous System. Cell. 174, 999-1014.e22 (2018).
33. D. Qin, J. Wang, A. Le, T. J. Wang, X. Chen, J. Wang, Traumatic Brain Injury: Ultrastructural Features in Neuronal Ferroptosis, Glial Cell Activation and Polarization, and Blood-Brain Barrier Breakdown. Cells. 10 (2021), doi:10.3390/cells10051009.
34. S. Fumagalli, F. Fiordaliso, C. Perego, A. Corbelli, A. Mariani, M. De Paola, M. G. De Simoni, The phagocytic state of brain myeloid cells after ischemia revealed by superresolution structured illumination microscopy. J. Neuroinflammation. 16, 1-14 (2019).
35. S. Z. Wang, Z. H. Qin, Anti-inflammatory and immune regulatory actions of Naja naja atra venom. Toxins (Basel). 10, 1-14 (2018).
36. D. Kunz, S. Schmitz, R. Mahlberg, A. Mohr, C. Stöter, K. J. Wolf, W. M. Herrmann, A new concept for melatonin deficit: On pineal calcification and melatonin excretion. Neuropsychopharmacology. 21, 765-772 (1999).
37. D. X. Tan, B. Xu, X. Zhou, R. J. Reiter, Pineal calcification, melatonin production, aging, associated health consequences and rejuvenation of the pineal gland. Molecules. 23 (2018), doi:10.3390/molecules23020301.
38. O. A. Ajijola, J. J. Wisco, H. W. Lambert, A. Mahajan, E. Stark, M. C. Fishbein, K. Shivkumar, Extracardiac Neural Remodeling in Humans With Cardiomyopathy. Circ. Arrhythmia Electrophysiol. 5, 1010-1116 (2012).
39. O. A. Ajijola, D. B. Hoover, T. M. Simerly, T. C. Brown, J. Yanagawa, R. M. Biniwale, J. M. Lee, A. Sadeghi, N. Khanlou, J. L. Ardell, K. Shivkumar, Inflammation, oxidative stress, and glial cell activation characterize stellate ganglia from humans with electrical storm. JCI Insight. 2, 1-11 (2017).
40. A. Lymperopoulos, G. Rengo, W. J. Koch, Adrenergic nervous system in heart failure: Pathophysiology and therapy. Circ. Res. 113, 739-753 (2013).
41. H. Sun, A. M. Gusdon, S. Qu, Effects of melatonin on cardiovascular diseases: Progress in the past year. Curr. Opin. Lipidol. 27, 408-413 (2016).
42. T. A. Wynn, K. M. Vannella, Macrophages in Tissue Repair, Regeneration, and Fibrosis. Immunity. 44, 450-462 (2016).
43. A. K. Dreismann, M. E. McClements, A. R. Barnard, E. Orhan, J. P. Hughes, P. J. Lachmann, R. E. MacLaren, Functional expression of complement factor I following AAV-mediated gene delivery in the retina of mice and human cells. Gene Ther. 28, 265-276 (2021).
44. M. Maier, Y. Peng, L. Jiang, T. J. Seabrook, M. C. Carroll, C. A. Lemere, Complement C3 deficiency leads to accelerated amyloid β plaque deposition and neurodegeneration and modulation of the microglia/macrophage phenotype in amyloid precursor protein transgenic mice. J. Neurosci. 28, 6333-6341 (2008).
45. M. Absinta, D. Maric, M. Gharagozloo, T. Garton, M. D. Smith, J. Jin, K. C. Fitzgerald, A. Song, P. Liu, J.-P. Lin, T. Wu, K. R. Johnson, D. B. McGavern, D. P. Schafer, P. A. Calabresi, D. S. Reich, A lymphocyte-microglia-astrocyte axis in chronic active multiple sclerosis. Nature. 597 (2021), doi: 10.1038/s41586-021-03892-7.
46. M. Gavriilaki, V. K. Kimiskidis, E. Gavriilaki, Precision medicine in neurology: The inspirational paradigm of complement therapeutics. Pharmaceuticals. 13, 1-25 (2020).
47. F. Matheis, P. A. Muller, C. L. Graves, I. Gabanyi, Z. J. Kerner, D. Costa-Borges, T. Ahrends, P. Rosenstiel, D. Mucida, Adrenergic Signaling in Muscularis Macrophages Limits Infection-Induced Neuronal Loss. Cell. 180, 64-78.e16 (2020).

## Claims

1. An anti-inflammatory agent for use in a method for inhibiting and/or preventing damage of a peripheral ganglion in a subject.

2. An anti-inflammatory agent for the use of claim 1, wherein the damage comprises degeneration and/or death of cells in said peripheral ganglion, particularly degeneration and/or death of neuronal cells and optionally degeneration and/or death of glial cells and/or fibrotic scarring.

3. An anti-inflammatory agent for the use of claim 1 or 2, wherein the damage comprises fibrotic remodeling of the peripheral ganglion.

4. An anti-inflammatory agent for the use of any one of claims 1-3, wherein the subject is suffering from a disorder associated with and/or accompanied by degeneration and/or cell death in a peripheral ganglion.

5. An anti-inflammatory agent for the use of claim 4, wherein the subject is suffering from a cardiac disease, particularly selected from selected from hypertension, myocardial infarction, congestive heart failure, cardiac arrhythmia, coronary artery disease, or pressure overload-induced cardiac dysfunction.

6. An anti-inflammatory agent for the use of any one of claims 4-5, wherein the subject is suffering from an acute form of the disorder.

7. An anti-inflammatory agent for the use of claim 6, wherein the subject is suffering from an acute myocardial infarction, and wherein the anti-inflammatory agent is administered in addition to a standard treatment, particularly to a revascularization treatment, e.g., a revascularization treatment of the posterior cerebral artery (PCA).

8. An anti-inflammatory agent for the use of any one of claims 1-7, wherein the anti-inflammatory agent is administered as a preventive intervention.

9. An anti-inflammatory agent for the use of any one of claims 1-8, wherein the peripheral ganglion is the superior cervical ganglion (SCG).

10. An anti-inflammatory agent for the use of any one of claims 1-9, wherein the anti-inflammatory agent is locally administered to the peripheral ganglion, e.g., by injection.

11. An anti-inflammatory agent for the use of any one of claims 1-10, wherein the anti-inflammatory agent is selected from:
- a glucocorticoid;
- an immunoglobulin preparation;
- a complement inhibitor;
- a nucleic acid therapeutic;
- an immunomodulator;
- an antagonist of a chemokine, cytokine or colony-stimulating factor; and
- a macrophage inhibitor.

12. A method for identifying a subject being at risk of damage of a peripheral ganglion, comprising determining at least one parameter selected from a physical parameter such as periodic repolarization dynamics and ganglion size, and/or a physiological parameter such as ganglion function, inflammation and/or a biomarker, e.g., melatonin, a biomarker generally associated with a cardiac disease, and/or a biomarker specifically associated with damage of a peripheral ganglion.

13. A compound which is a modulator of a biomarker selected from a gene which is **characterized by** an altered, e.g., increased, or decreased expression level associated with damage or risk of damage in a peripheral ganglion, e.g., the SCG, for use in a method for preventing damage of a peripheral ganglion in a subject.

14. The compound of claim 13 for the use of claim 13 which is an agonist of the biomarker or an antagonist of the biomarker.

15. The compound of any one of claims 13-14 for the use of claim 13, which is selected from an antibody or an antigen-binding fragment thereof and a nucleic acid molecule, e.g., an mRNA molecule, an antisense molecule or a siRNA molecule.
